# EUROPEAN PATENT SPECIFICATION

(11) **EP 2 066 794 B1**
(45) Date of publication and mention of the grant of the patent: **05.01.2011**
(21) Application number: 07803195.2
(22) Date of filing: 04.09.2007
(51) Int. Cl.: C12N 15/85

(54) **ANIMAL MODEL FOR THE SELECTION AND VALIDATION OF AGENTS ACTIVE AGAINST PULMONARY EMPHYSEMA AND COLORECTAL CANCER**
TIERMODELL ZUR AUSWAHL UND VALIDIERUNG VON GEGEN LUNGENEMPHYSEM UND KOLOREKTALKARZINOM WIRKSAMEN AGENTIEN
MODÈLE ANIMAL PERMETTANT LA SÉLECTION ET LA VALIDATION D'AGENTS ACTIFS CONTRE L'EMPHYSÈME PULMONAIRE ET LE CANCER COLORECTAL

(30) Priority: 15.09.2006 EP 06120714
(43) Date of publication of application: 10.06.2009
(73) Proprietor: FrankGen Biotechnologie AG, 61476 Kronberg/Taunus (DE)
(72) Inventor: MELCHNER, Harald von, 61476 Kronberg (DE); WEMPE, Frank, 63165 Mühlheim Main (DE); DE-ZOLT, Silke, 63517 Rodenbach (DE)
(74) Representative: von Kreisler Selting Werner
(86) International application number: PCT/EP2007/059227
(87) International publication number: WO 2008/031746

(56) References cited:
- WO-A-2006/056340
- WO-A2-03/015505
- BUDANOV ANDREI V ET AL: "Regeneration of peroxiredoxins by p53-regulated sestrins, homologs of bacterial AhpD" SCIENCE (WASHINGTON D C), vol. 304, no. 5670, 23 April 2004 (2004-04-23), pages 596-600, XP002410471 ISSN: 0036-8075 cited in the application
- BOZONET STEPHANIE M ET AL: "Oxidation of a eukaryotic 2-Cys peroxiredoxin is a molecular switch controlling the transcriptional response to increasing levels of hydrogen peroxide" JOURNAL OF BIOLOGICAL CHEMISTRY, vol. 280, no. 24, June 2005 (2005-06), pages 23319-23327, XP002410472 ISSN: 0021-9258
- DONADELLI ET AL: "Increased stability of P21<WAF1/CIP1> mRNA is required for ROS/ERK-dependent pancreatic adenocarcinoma cell growth inhibition by pyrrolidine dithiocarbamate" BIOCHIMICA ET BIOPHYSICA ACTA. MOLECULAR CELL RESEARCH, ELSEVIER SCIENCE PUBLISHERS, AMSTERDAM, NL, vol. 1763, no. 9, 6 June 2006 (2006-06-06), pages 917-926, XP005647103 ISSN: 0167-4889
- GRANBERG ET AL: "Adenovirus-induced alterations in host cell gene expression prior to the onset of viral gene expression" VIROLOGY, ACADEMIC PRESS,ORLANDO, US, vol. 353, no. 1, 24 July 2006 (2006-07-24), pages 1-5, XP005614449 ISSN: 0042-6822
- KANG ET AL: "2-Cys peroxiredoxin function in intracellular signal transduction: therapeutic implications" TRENDS IN MOLECULAR MEDICINE, ELSEVIER CURRENT TRENDS, vol. 11, no. 12, December 2005 (2005-12), pages 571-578, XP005193381 ISSN: 1471-4914
- SABLINA ANNA A ET AL: "The antioxidant function of the p53 tumor suppressor" NATURE MEDICINE, vol. 11, no. 12, December 2005 (2005-12), pages 1306-1313, XP002410599 ISSN: 1078-8956 cited in the application

## Description

The present invention relates to a rodent animal model, cell and tissue cultures derived therefrom, which do not produce or produce only suboptimal levels of one or more functional sestrins and in addition do not produce or do only produce suboptimal levels of latent transforming growth factor β binding protein 4 (ltbp4). Furthermore, the present invention relates to a method for selecting agents for treating the pulmonary emphysema and/or the colorectal cancer exhibited by utilizing the animal model, cell or tissue culture of the invention. The rodent animal model, cell or tissue culture is suitable for preclinical testing of efficacy, toxicity and bioavailability of potential agents.

### Background of the Invention

Chronic obstructive lung disease with pulmonary emphysema (COPD) is a highly prevalent disease that has a large impact on quality of life for patients and their families and kills millions of people worldwide. While the major risk factor for COPD is the inhalation of toxic gases and particles that are primarily but not exclusively generated in tobacco smoke, primary (genetic) airway abnormalities are also involved as only a fraction of smokers develop COPD. COPD is associated with major healthcare costs, largely due to expensive treatments such as long-term oxygen therapy and hospital admissions, as well as indirect costs including loss of working capacity. Moreover, treatment of COPD is limited to symptoms as no pharmacologic intervention has been shown so far to modify the natural history of the disease (Vestbo J. and Hogg, J. C. Thorax. 61:86-8, 2006; Fabbri L. M., et al. Am J Respir Crit Care Med. 173:1056-65, 2006).

Colorectal cancer is one of the most common neoplasms in the Western hemisphere and represents a major public health challenge despite progress in detection and therapy. The overall mortality at 5 years is about 40%. While early stage disease can be cured by surgery, only palliative treatment is available for patients with non-resectable, metastatic cancer whose survival rate is between 7-24 months (Shaheen N. J., et al. Am J Gastroenterol. 2006).

From the above in becomes apparent that new pharmaceutical compounds for the treatment of both conditions are highly desirable and could be identified with the animal model of the invention, which exhibits both pulmonary emphysema and colorectal cancer as a result of loss of function mutations in the sestrin 2- and the latent transforming growth factor beta binding protein 4 genes.

Transforming growth factor-βs (TGF-βs) belong to a protein superfamily whose members control cell growth and differentiation in a variety of adult tissues and are involved in a wide range of immune and inflammatory responses (Shi Y. and Massague, J. Cell. 113:685-700, 2003; Sheppard D. Proc Am Thorac Soc. 3:413-7, 2006). Most cells secrete TGF-β as a functionally inactive, latent cytokine complexed to its latency associated propeptide (LAP), which interacts with its receptors only after activation by proteolytic cleavage. There are two known, structurally different latent complexes of TGF-β. The small latent complex consists of a mature TGF-β dimer bound to the N-terminal portion of the TGF-β-LAP propeptide. The large latent complex contains, in addition to TGF-β-LAP, one of the three latent TGF-β binding proteins (ltbps 1, 3 and 4). Unless displaced from the ltbps after secretion, TGF-β is usually deposited in the extracellular matrix (ECM) as a high molecular weight complex with ltbp and LAP (Annes J. P., et al. J Cell Sci. 116:217-24, 2003). All 4 existing isoforms of ltbp (ltbp 1-4) have been mutationally inactivated in the mouse (Dabovic B., et al. J Cell Biol. 56:227-232, 2002; Sterner-Kock A., et al. Genes Dev. 16:2264-2273, 2002; Shipley J. M., et al. Mol Cell Biol. 20:4879-87, 2000). While the phenotypes of the knock out mice varied among isoforms, both ltbp3 and ltbp4 mutants develop pulmonary emphysema due to defects in TGF-β signaling. However, in contrast to the emphysema developing mutant ltbp3 mice, which is relatively mild and strictly developmental (Dabovic B., et al. J Cell Biol. 56:227-232, 2002), emphysemas in mutant ltbp4 (*ltbp4*^{*-*/*-*}) mice worsen with age and progressively acquire the features of late onset emphysemas that develop in the context of COPD in humans (Sterner-Kock A., et al. Genes Dev. 16:2264-2273, 2002; WO 03/015505). The potential relevance of ltbp4 in the pathogenesis of COPD has been recently underscored by a clinical study reporting a highly significant correlation between specific COPD symptoms and a single nucleotide polymorphism (SNP) in *ltbp4* (Hersh C. P., et al. Am J Respir Crit Care Med. 173:977-84, 2006). In addition to the emphysema, mutant *ltbp4* mice develop invasive colorectal cancer (Sterner-Kock A., et al. Genes Dev. 16:2264-2273, 2002; WO 03/015505). A link between *ltbp4* expression and cancer progression has also been recently reported in a clinical study of patients with colon cancer (Bertucci F., et al. Oncogene. 23:1377-91, 2004).

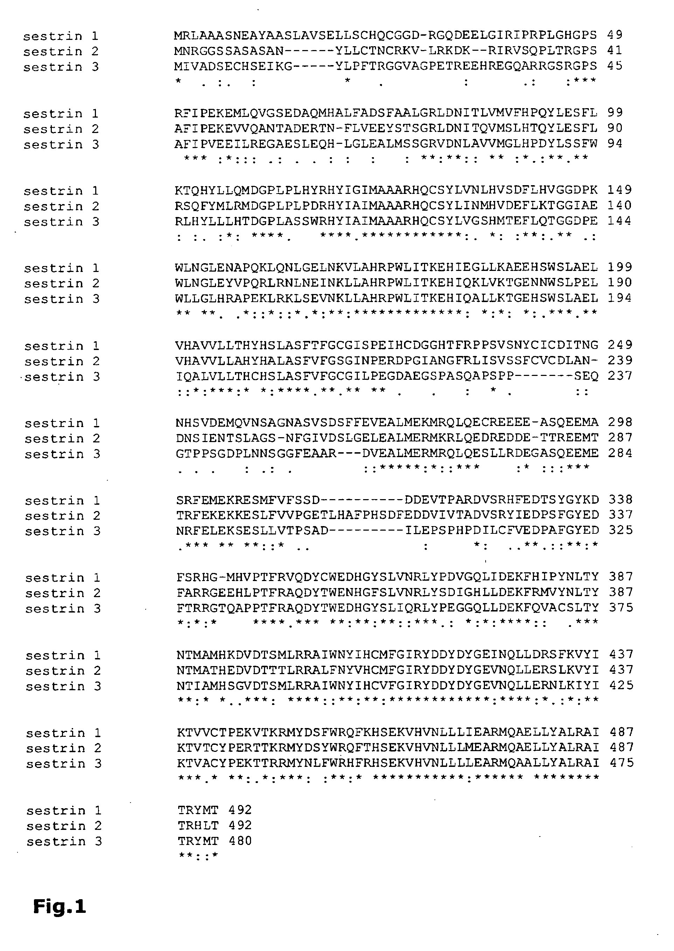

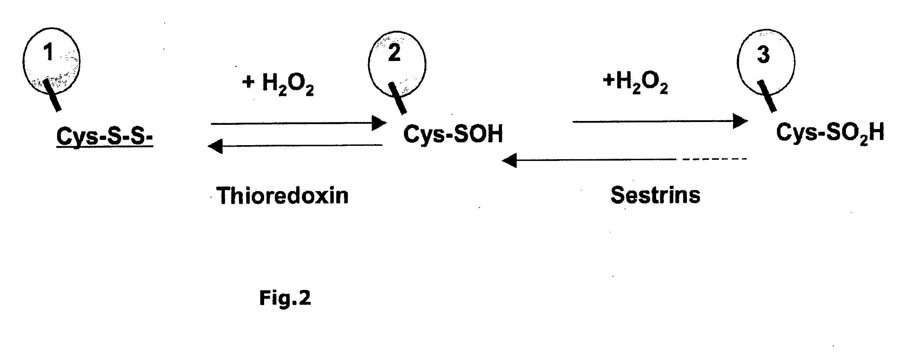

Sestrin 2 belongs to a family of highly conserved proteins initially discovered as p53 inducible proteins (Velasco-Miguel S., et al. Oncogene. 18:127-37, 1999; Peeters H., et al. Hum Genet. 112:573-80, 2003; Budanov A. V., et al. Oncogene. 21:6017-31, 2002; WO 00/12525; US 2002/0103353). Mammalian cells express three isoforms referred to as sestrin 1 (sestrin 1; also known as PA26), sestrin 2 (sestrin 2; also known as Hi95) and sestrin 3 (Figure 1). Two of the sestrins (sestrin 1 and sestrin 2) have been recently shown to regulate intracellular peroxide (ROS) levels (Budanov A. V., et al. Science. 304:596-600, 2004; Sablina A. A., et al. Nat Med. 11:1306-13, 2005). More specifically, sestrins are believed to regenerate (reduce) peroxiredoxins (Prxs), which are highly conserved and ubiquitously expressed, antioxidant proteins (reviewed in Georgiou G. and Masip, L. Science. 300:592-4, 2003; Wood Z. A., et al. Trends Biochem Sci. 28:32-40, 2003). While reducing H₂O₂ to H₂O, Prxs are oxidized at their so called peroxidatic cystein (Cys-SH), which is converted into sulfenic acid (Cys-SOH) (Figure 2). In bacteria, where the Prx AhpC is the primary H₂O₂ scavenger, the oxidized AhpC is subsequently reduced by the dedicated reductase AhpF. However, unlike AhpC, mammalian Prxs are highly susceptible to overoxidation yielding sulfinic acid (Cys-SO₂H) in presence of high peroxide concentrations (Figure 2). Protein sulfinic acids cannot be reduced by typical cellular reductants such as glutathione and thioredoxin and therefore their formation has been considered an irreversible process. Once Prx has been converted to sulfinic acid it is no longer enzymatically active and inactive enzyme accumulates in cells exposed to oxidative stress (Wood Z. A., et al. Trends Biochem Sci. 28:32-40, 2003). However, more recently gradual recovery of Prxs has been observed after initial oxidative inactivation in eukaryotic cells (Mitsumoto A., et al. Free Radic Biol Med. 30:625-35, 2001; Woo H. A., et al. Science. 300:653-6, 2003) and sestrins have been proposed as the catalyzing enzymes due to their homology to the bacterial sestrin homolog -AhpD-, which regenerates the AhpC Prx and due to their ability to reduce intracellular ROS levels (Budanov A. V., et al. Science. 304:596-600, 2004).

In eukaryotic cells Prxs are believed to be both antioxidants protecting against stress and regulators of ROS-mediated signaling (Wood Z. A., et al. Science. 300:650-3, 2003) It has been known for some time that receptor/ligand interactions generate bursts of ROS, which act as second messengers in signal transduction pathways, including the TGF-β pathway (Bae Y. S., et al. J Biol Chem. 272:217-21, 1997; Lo Y. Y. and Cruz, T. F. J Biol Chem. 270:11727-30, 1995; Mills E. M., et al. J Biol Chem. 273:22165-8, 1998; Sundaresan M., et al. Science. 270:296-9, 1995; Thannickal V. J., et al. J Biol Chem. 273:23611-5, 1998; Thannickal V. J., et al. Faseb J. 14:1741-8, 2000). Defective TGF-β signaling has been shown to be involved in the pathology of pulmonary emphysema and colorectal cancer in both animal models (including WO 03/015505 A3) and men (Morris D. G., et al. Nature. 422:169-73, 2003; Neptune E. R., et al. Nat Genet. 33:407-11, 2003; Sterner-Kock A., et al. Genes Dev. 16:2264-2273, 2002; Massague J., et al. Cell. 103:295-309, 2000; Zhu Y., et al. Cell. 94:703-14, 1998).

The association between ROS and TGF-β is complex and occurs at different levels of the signaling cascade. First, ROS have been shown to activate latent TGF-β1 both in vitro and in vivo (Annes J. P., et al. J Cell Sci. 116:217-24, 2003; Barcellos-Hoff M. H. and Dix, T. A. Mol Endocrinol. 10:1077-83, 1996). In line with this, Fatma et al. have recently reported that lens epithelial cells derived from Prx6 knock out mice are highly susceptible to oxidative stress and develop a phenotype indistinguishable from TGF-β stimulation. They could attribute this phenotype to a ROS mediated activation of latent TGF-β, which was readily reversible by antioxidants (Fatma N., et al. Cell Death Differ. 12:734-50, 2005). Second, TGF-β requires ROS for the induction of various target genes such as PAI-1, CTGF and extracellular matrix genes (Jiang Z., et al. Biochem Biophys Res Commun. 309:961-6, 2003; Park S. K., et al. Biochem Biophys Res Commun. 284:966-71, 2001). Recent studies have shown that TGF-β induced smad2,3 phosphorylation is also partly dependent on ROS (Cucoranu I., et al. Circ Res. 97:900-7, 2005) presumably via the inactivation of a dedicated phosphatase. It has been known for some time that phosphatases are susceptible to inactivation by ROS (Seo J. H., et al. Mol Biol Cell. 16:348-57, 2005; Chiarugi P. and Cirri, P. Trends Biochem Sci. 28:509-14, 2003) and a smad2,3 phosphatase (PPM1A) capable of terminating TGF-β signaling has been recently discovered (Lin X., et al. Cell. 125:915-28, 2006. Third, TGF-β itself induces superoxide production by activating NADPH oxidases presumably by the transcriptional upregulation of Nox4 (Sturrock A., et al. Am J Physiol Lung Cell Mol Physiol. 290:L661-L673, 2006).

### Description of the Invention

It was now found that the genetic ablation of a sestrin, notably sestrin 2, from the animal model (*ltbp4*^{*-*/}*⁻* mice model) described in WO 03/015505 resulted in a significant recovery of the disease conditions (see examples), indicating that this family of proteins could provide molecular targets for the treatment of COPD and colorectal cancer. It is believed that ablation of sestrin 2 in the animal model of WO 03/015505, reactivated TGF-β signaling, presumably by increasing intracellular second messenger ROS levels. This in turn significantly improved both disease conditions as illustrated by the examples, suggesting that pulmonary emphysema and colon cancer can be treated by antagonizing sestrin function. The present invention thus relates to
(1) a rodent animal model, which does not produce one or more functional sestrins or produces translational levels reduced at least by 50% of one or more sestrins, preferably sestrin 2 and in addition does not produce latent transforming growth factor β binding protein 4 (hereinafter "ltbp4") or produces translation levels reduced at least by 50% of ltbp4;
(2) a rodent animal model, which does not express one or more functional sestrins or expresses translational levels reduced at least by 50% of one or more sestrins;
(3) a cell or tissue culture isolated from the rodent animal models as defined in (1) or (2) above, wherein levels of expression, in the cell or in the cells of the tissue, of the sestrin and ltbp 4 proteins are as defined in (1) or (2);
(4) a method for preparing the rodent animal model of (1) above, which comprises disrupting the sestrin and/or ltbp4 gene in a germ cell of a starting rodent animal;
(5) a method for selecting an agent for treating a symptom occurring in the rodent animal model of (1) above comprising:
   (i) applying one or more agents to be tested to said rodent animal model,
   (ii) determining whether one or more symptoms occurring in said rodent animal model have changed as a result of application of said agent(s);
(6) a method selecting an agent that interferes with ROS production and TGF-β signaling comprising:
   (i) applying one or more agents to be tested to the to the cell or tissue culture of (3) above,
   (ii) determining whether cellular ROS levels and TGF-β signaling have changed as a result of the application of said agents or agents;
(7) a method to analyze whether cancer and/or pulmonary emphysema is caused by differential ltbp4 and sestrin gene or protein expression or expression level or by a defect in the ltbp4 and sestrin gene comprising:
   (i) characterizing the ltbp4 and sestrin gene or protein expression or expression level or ltbp4 and sestrin gene allele status of an individual having cancer or pulmonary emphysema,
   (ii) characterizing the ltbp4 and sestrin gene or protein expression or expression level or ltbp4 and sestrin gene allele status of a control individual, a difference in the ltbp4 and sestrin gene or protein expression or expression level or ltbp4 and sestrin gene allele status indicating that cancer and/or pulmonary emphysema and/or cardiomyopathy is linked to differential ltbp4 and sestrin gene or protein expression or expression level or a defect in the ltbp4 and sestrin genes;

In conclusion, the present invention provides a rodent animal model of human disease that reveals some crucial functions of the sestrin proteins. In view of these functions, the rodent animal model of the invention can be used to develop novel treatments for pulmonary emphysema and cancer that target sestrin proteins and their role in ROS metabolism and TGF-β signaling.

### Description of the Figures

Figure 1: Amino acid sequence of the mouse sestrins and homology between isoforms. The sequences of the isoforms are further shown in SEQ ID NOs:1 to 3.
Figure 2: Oxidation of the peroxidatic cystein of peroxiredoxins (Prxs) by superoxides. Reduced Prxs form dimers via a disulfide bond (1). Oxidized Prxs form sulfenic acid (2), which is reduced by thioredoxin or glutathion in a reverse reaction (1). Overoxidized Prxs form sulfinic acid, which cannot be reduced by thioredoxin or glutathion (3). Sestrins are believed to regenerate the overoxidized Prxs. For further explanation see text.
Figure 3: pT1βgeo gene trap insertion in the last intron of the sestrin 2 gene (Ensembl Id: *ENSMUSG00000028893*) and position of the allele specific primers (arrows). The resulting fusion protein lacks 27 aa of sestrin 2.
Figure 4: Whole mount E7.5 embryo stained with X-Gal.
Figure 5: Analysis of sestrin 2 gene expression in W077E04 mutant mice. A. qRT-PCR of total RNA extracted from lungs of wild type (WT), heterozygous (+/-) and homozygous (-/-) mice. B. Western blot analysis of sestrin 2 protein expression in isolated mouse colon (lanes 1, 2) and lung (lanes 3, 4) fibroblasts using polyclonal anti-mouse sestrin 2 (PTG Inc., Chicago, IL) and anti-tubulin antibodies. Minor bands reacting with polyclonal anti-sestrin 2 antibody are
most likely non-specific.
Figure 6: Tracheobronchial silicone casts of 5.5 months old wild type, single ltbp4 and double *ltbp4*/*sesn2* mutant mice.
Figure 7: Pulmonary emphysema of age matched single *ltbp4* double *ltbp4*/*sesn2* mutant mice. HE stain at x 40 magnification.
Figure 8: Elastin and collagen content in single *ltbp4* and double *ltbp4*/*sesn2* mutant lungs. A. Elastin (black) stained with Weigert's resorcin-fuchsin at x 200 magnification. B. collagen (blue) stained with Masson's trichrome stain at x 200 magnification.
Figure 9: Tracheobronchial silicone cast of an *ltbp4*^{-/-} lung heterozygous for *sesn2* null alleles.
Figure 10: Collagen deposition in *ltbp4* mutant lungs heterozygous (*sesn*^{+/-}) and homozygous (*sesn*^{*-l*-}) for the *sesn2* null allele. Collagen (blue) stained with Masson's trichrome stain at x 200 magnification.
Figure 11: Colorectal adenoma in age matched single (*ltbp4*^{-/-}) and double (*ltbp4*^{-/-} *sesn2*^{-/-}) mutant mice.
Figure 12: P-smad2 levels in the colon of age matched single (*ltbp4*^{-/-}) and double (*ltbp4*^{-/-} *sesn2*^{-/-}) mutant mice. Positive cells are reddish-brown.
Figure 13: P-smad2 levels in *sesn2* mutant lungs. Lung section were stained with polyclonal anti-P-smad2 antibody. Positive cells are reddish-brown.
Figure 14: Increased connective tissue deposition in mutant *sesn2* lungs. HE stain at x 200 (top) and x 400 (bottom) magnifications.
Figure 15: ROS levels in MLFs. A. Basal and H₂O₂-induced (200 µM, 3 hours) ROS amounts measured by FACS (530 nm) after DCF treatment. FL1-H, fluorescence intensity. B. Basal and H₂O₂ induced ROS amounts accumulating in MLFs expressed as mean intensity of cell fluorescence ± SD of the two independent experiments shown in A.

### Detailed Description of the Invention

The particular terms and abbreviations utilized to define the present invention are further defined in the following.

The terms "does not produce functional sestrins" and "does not produce functional ltbp4" denote the lack of effective sestrin and ltbp4 expression, respectively. A "lack of effective sestrin and ltbp4 expression" also includes the expression of non-functional (i.e. truncated) sestrins and ltbp4 proteins which are not exerting the function of the native protein.

The term "produces suboptimal levels of sestrins" and "produces suboptimal levels of ltbp4" encompasses the translation level of sestrin and ltbp4 proteins, which are insufficient to exert their function. Preferably, the level is reduced at least by 50%, more preferably by 70% and most preferably by 100 %.

The term "homozygous disruption" relates to an identical mutation in both alleles of a gene.

The term "heterozygous disruption" relates to a mutation in only one allele of gene. The term "mutation" refers to a change of one or more nucleotide pairs of a DNA molecule.

The term "insertion" is directed to a mutation identified by the presence of one or more additional base pairs in the DNA. The term "deletion" relates to a mutation generated by removal of a sequence of DNA (one or more base pairs), the regions on either side being joined together. The term "substitution mutation" is directed to a nucleotide exchange. The substitution mutation can result in an amino acid change or can introduce a premature translation stop codon. Furthermore, a substitution mutation can affect splicing or expression of the gene when occurring at sites necessary for splicing or gene regulation.

The term "gene targeting" relates to a type of homologous recombination that occurs when a fragment of genomic DNA is introduced into a cell and that fragment recombines with homologous sequences in the genome. The term "gene trap integration" is directed to insertion of a vector, which comprises a reporter gene and which is activated upon insertion into an active transcription unit of the genome. The term "mutagenesis" denotes a chemical or physical treatment that changes nucleotides in the genome of an organism. An example of a chemical mutagenesis is N-ethyl-N-nitrosurea (ENU) mutagenesis.

The term "exon" encompasses a segment of a gene, which is decoded to give a mRNA product. Individual exons may contain protein coding DNA and/or noncoding DNA. The term "intron" denotes non-coding DNA, which separates neighboring exons in a gene. During gene expression, introns like exons are transcribed into RNA but the transcribed intron sequences are subsequently removed by RNA splicing and are not present in mRNA. The term "regulatory region" relates to the nucleotide sequence, which comprises regions that are necessary for the regulation of gene transcription. These regions comprise, for example, promoters and enhancers and they can be located in 5' untranslated regions, exons, introns and 3'UTRs. The term "splice site" encompasses the nucleotides at the beginning and the end of the intron that are required for the joining of two exons by removing the intercalated intron during primary transcript processing to functional mRNA.

The term "pulmonary emphysema" denotes a symptom of chronic obstructive lung disease (COPD) characterized by a size increase beyond normal of air spaces distal to the terminal bronchioles and inflammatory infiltrates.

The term "cardiomyopathy" designates a primary non-inflammatory disease of the heart muscle, which is the result of pulmonary hypertension that complicates COPD. The term "cancer" refers to an uncontrolled proliferation of epithelial cells lining the colonic crypts.

"ROS metabolism" refers to the production and neutralization (reduction) of intracellular reactive oxygen species such as hydrogen peroxide and oxygen anions. The term "profibrotic changes" refers to an increased tissue deposition of collagen associated with a multiplication of fibroblasts.

The term "selecting an agent for treating a symptom" encompasses choosing a composition for management of the condition.

The term "application of one or more agents" relates to administering single compounds or compound combinations orally, by inhalation, parenterally, e.g. intravenously, subcutaneously, intraperitoneally or intramuscularly, or topically, e.g. ophtalmically, vaginally, rectally or intranasally.

The invention is hereinafter described in more detail by referring to the accompanying Figures and Examples.

In a preferred embodiment of aspect (1) of the invention, the genome of the rodent comprises a homozygous disruption of the *sesn2* and *ltbp4* genes. Preferably this homozygous disruption has been generated by a mutation and this mutation can be an insertion, deletion or a substitution mutation. Furthermore, preferably said mutation is generated by gene targeting, gene trapping or chemical mutagenesis and it has occurred in an exon, intron, regulatory region or splice site of the sestrin 2 gene, preferably into the last intron (i.w. the 9^{th} intron) of the sestrin 2 gene, and in an exon, intron, regulatory region or splice site of the ltbp4 gene, preferably in the 5^{th} intron of *ltbp4* . It is also preferred that the mutation sites rise to an expression of a detectable reporter gene such as fluorescent proteins (such as GFP and its derivatives), enzymes (such as LacZ) or selection markers (such as βgeo). Particularly preferred is that
(i) the sestrin 2 gene is disrupted in the 9^{th} intron by inserting a gene trap vector, preferably pT1βgeo (SEQ ID NO:10); and/or
(ii) the ltbp4 gene is disrupted in the 5^{th} intron by inserting a gene trap vector, preferably U3Cre (SEQ ID NO:16).

In a further preferred embodiment said rodent comprises a heterozygous disruption of the sens2 gene and a homozygous disruption of ltbp4 gene or homozygous disruption of the sestrin 2 gene and a heterozygous disruption of the ltbp4 gene.

In another aspect of the invention the rodent animal model exhibits pulmonary emphysema and/or cardiomyopathy and/or colorectal cancer. Furthermore, the rodent is preferably a mouse or rat.

In a further preferred embodiment the rodent animal model exhibits defects in ROS metabolism and/or profibrotic changes in one or more major organs, peferably in the lung or colon.

In a further preferred embodiment the rodent animal model develops symptoms that are less severe than those exhibited by the animal model disclosed in WO 03/015505.

Embodiment (3) of the invention relates to a cell or tissue culture isolated from a rodent animal model of (1) or (2). Preferably, the cell is derived from the lung or colon.

In a preferred aspect of this embodiment, the cell or tissue culture is isolated from a rodent animal model whose genome comprises a homozygous disruption of one or more sestrin genes such that said genes do not produce functional sestrins, preferably sestrin 2 and a homozygous disruption of the ltbp4 gene such that said gene does not produce functional ltbp4.

In another preferred aspect of this embodiment, the cell or tissue culture is isolated from a rodent animal model whose genome comprises a heterozygous disruption of one or more sestrin genes such that said genes do produce only 50% or less of functional sestrins, preferably sestrin 2 and a homozygous disruption of the ltbp4 gene such that said gene does not produce functional ltbp4.

In another preferred aspect of this embodiment, the cell or tissue culture is isolated from a rodent animal model whose genome comprises a homozygous disruption of one or more sestrin genes, preferably sestrin 2 and a heterozygous disruption of *ltbp4* such that said gene produces only 50% or less of functional ltbp4. Particularly preferred is that the cell or tissue culture is derived from lung or colon. Embodiment (4) of the invention relates to a method for preparing the rodent animal model of embodiments (1) and (2), which comprises disrupting the sestrin and/or ltbp4 gene in a germ cell of a starting non-human animal. In a preferred aspect of this embodiment, the germ cell is an ES cell. The method may further comprise introducing the resulting ES cells into blastocysts, injecting the obtained blastocysts into respective rodent foster mothers and intercrossing the resulting chimeras.

Embodiment (5) of the invention is directed to a method for selecting an agent or agents for treating a symptom occurring in the rodent animal model of the invention comprising: (i) applying one or more agents to be tested to the animal model of the invention; and (ii) determining whether one or more symptoms occurring in the rodent animal model of the present invention have changed as a result of the application of said agent or agents. In a preferred embodiment the symptom is selected from a group consisting of pulmonary emphysema, cardiomyopathy and cancer. The agent, which is suitable for treating a symptom occurring in the rodent animal model of the invention, may be a pharmaceutical. The agent, which is suitable for treating a symptom occurring in the animal model of the invention may be suitable for the preparation of a pharmaceutical composition for the treatment of pulmonary emphysema.

Embodiment (6) of the invention is directed to a method selecting an agent that interferes with ROS production and TGF-β signaling comprising:
(i) applying one or more agents to be tested to the to the cell or tissue culture of (3) above,
(ii) determining whether cellular ROS levels and TGF-β signaling have changed as a result of the application of said agents or agents.

It is particularly preferred in said method that the cell or tissue culture is from lung or colon.

Embodiment (7) of the invention is a method to analyze whether pulmonary emphysema and/or cancer is caused by differential ltbp4 and sestrin gene or protein expression or expression level or by a defect in the ltbp4 and sestrin genes comprising the (i) characterization of the ltbp4 and sestrin gene or protein expression or expression level or of the ltbp4 and sestrin gene allele status of an individual having pulmonary emphysema and/or cancer, and the (ii) characterization of the ltbp4 and sestrin gene or protein expression or expression level or ltbp4 and sestrin gene allele status of a control individual. A difference in the ltbp4 and sestrin gene or protein expression or expression level or in the ltbp4 and sestrin gene allele status indicates that a defect in the ltbp4 and sestrin genes is involved in the pathogenesis of pulmonary emphysema and/or cancer.

The method described in (7) may be used for diagnosing pulmonary emphysema or cancer comprising the (i) characterization the ltbp4 and sestrin gene or protein expression or expression level or the ltbp4 and sestrin gene allele status of an individual and (ii) characterization of the ltbp4 gene or protein expression or expression level or ltbp4 and sestrin gene allele status of a control individual. A difference in the ltbp4 and sestrin gene or protein expression or expression level or in the ltbp4 and sestrin gene allele status would indicate the presence of pulmonary emphysema and/or cancer in said individual. Among the determined expression levels it is the ltbp4 expression level that is an indicator of the severity of the pulmonary emphysema or of the cancer, while the sestrin expression level is a marker for the disease progression such that low expression levels are beneficial to the disease progression.

"Individual" used in connection with the embodiment (7) relates to an individual with suspected abnormal ltbp4 and sestrin gene allele status, i.e. a patient. "Control individual" refers to a healthy individual having normal ltbp4 and sestrin allele status.

In a preferred embodiment of the methods to analyze whether pulmonary emphysema and/or cancer are linked to ltbp4 and sestrin and for diagnosing pulmonary emphysema and cancer, the ltbp4 and sestrin gene allele status and the ltbp4 and sestrin expression or expression level are detected by genomic PCR, RT-PCR, Northern analysis, microarray (DNA chip) analysis or antibodies directed to the ltbp4 and sestrin proteins.

The methods of embodiments (5) to (7) are suitable to be performed *in vivo* and *in vitro.*

The rodent animal model of the present invention can be used to dissect the molecular mechanisms controlling the sestrin/TGF-β pathway and for the identification and cloning of modifier genes able to modify, aggravate, reduce or inhibit the phenotype associated with pulmonary emphysema and cancer or other conditions occurring in the animal model of the invention. Moreover, the rodent animal model can be used for identification of early diagnostic markers for cancer and/or pulmonary emphysema or other conditions occurring in the animal model of the invention. In addition, the rodent animal model of the present invention can be used for the monitoring of the activity of agents useful in the prevention or treatment of cancer and/or pulmonary emphysema or other conditions occurring in the rodent animal model of the invention and as a test model system for agents suspected of promoting or aggravating cancer and/or pulmonary emphysema or other conditions occurring in the animal model of the invention.

The invention furthermore explained in the following examples which are, however, not to be construed as a limitation of the invention.

### Examples

### Methods

Cell cultures: [129/SvPas] strain derived ES cells were grown on irradiated (32 Gy) MEF feeder layers in DMEM supplemented with 15% (v/v) preselected and heat inactivated fetal calf serum (FCS) (Linaris, Bettingen, Germany), 100 mM nonessential amino acids (Gibco), 0.1 mM mercaptoethanol (Sigma), 1000 U/ml of leukemia inhibitory factor (LIF) (Esgro^{R}; Gibco /BRL), as described (De-Zolt S., et al. Nucleic Acids Res. 34:e25, 2006). Lung and colon fibroblast cultures from adult wild-type and sens-/- mice were established according to standard protocols as previously described (Koli K., et al. J Cell Biol. 167:123-33, 2004) and grown in Dulbeccos's modified Eagle's medium (DMEM) supplemented with 10% fetal calf serum (Life Technologies), 100 IU/ml penicillin and 50 µg/ml streptomycin. During the first two weeks in culture spontaneous immortalization occurred generating cell lines.

5' RACE and sequencing: cDNAs from gene trap expressing ES cell lines were prepared from the polyadenylated RNA using a RoboAmp robotic device (MWG Biotech, Ebersberg, Germany) with a processing capacity of 96 samples/day. Samples of 2x10⁵ cells were lysed in 1 ml of lysis buffer containing 100 mM Tris/HCl pH 8.0, 500 mM LiCl, 10 mM EDTA, 1% LiDS and 5 mM DTT. Polyadenylated RNA was captured from the lysates by biotin-labeled oligo-d(T)-primers according to the manufacturers instructions (Roche Diagnostics Corp., Indianapolis, IN, USA) and placed on streptavidin-coated 96-well plates (AB Gene, Surrey, UK). After washing, solidphase cDNA synthesis was performed in-situ using random hexamers and SuperScript II RT (Invitrogen, Karlsruhe, Germany). To remove excess primers the cDNAs were filtered through Multiscreen PCR plates (Millipore Corp. Bedford, MA, USA). The 5' ends of the purified cDNAs were tailed with dCTPs using terminal transferase -TdT- (Invitrogen, Karlsruhe, Germany) following the manufacturer's instructions. For PCR amplification of the cellular sequences appended to the gene trap transcripts the following vector-specific primers were used: 5'-CTA CTA CTA CTA GGC CAC GCG TCG ACT AGT ACG GGI IGG GII GGG IIG-3' (SEQ ID NO:4) and 5'-GCC AGG GTT TTC CCA GTC ACG A-3' (SEQ ID NO:5);and for nested PCR: 5'-CTA CTA CTA CTA GGC CAC GCG TCG ACT AGT AC-3' (SEQ ID NO:6) and 5'-TGT AAA ACG ACG GCC AGT GTG AAG GCT GTG CGA GGC CG-3' (SEQ ID NO:7). Amplification products were directly sequenced using AB377 or ABI3700 sequencing machines (Applied Biosystems ABI, Foster City, USA).

ES cell injections, breeding and genotyping: W077E06 (TBV-2; 129SvPas) ES cell derived chimeras were generated by injecting C57Bl/6 blastocysts. The resulting male chimeras were bred to C57B1/6 females, and F1 agouti offspring containing the disrupted transgene were intercrossed to obtain homozygous F2 mice. Genotyping was performed on mouse tail DNA by genomic PCR using primers against the sequences flanking the gene trap insertion previously identified in ES cells by inverse PCR and sequencing.

Histology, histochemistry and immunohistochemistry: Paraffin sections of mouse tissues were prepared and stained using standard histology procedures. To visualize elastin and collagen fibers, microscopic slides were stained with Weigert's resorcin-fuchsin or Masson's trichrome stain as previously described (Sterner-Kock A., et al. Genes Dev. 16:2264-2273, 2002). Immunostainings using rabbit polyclonal anti- P-Smad2 antibodies were performed as described in Sterner-Kock et al (Sterner-Kock A., et al. Genes Dev. 16:2264-2273, 2002)

Plastinations: Tracheas of lungs prepared from wild-type and mutant mice were injected at room temperature with 1-2 ml of E RTV silicone (Dow Corning, Midland, MI, USA) using a 2 ml syringe. Curing Agent was added at a 1:10 ratio to the silicone polymere immediately prior to injection. Injection was stopped when the silicone was visible under the surface of both lungs. Following injection, the silicone was allowed to harden fro 24-48 hours after which the specimens were placed in 10% potassium hydroxide solution for 5-7 days. and then left in boiling water for 8-12 hours to detach the tissue from the polymer. Following maceration in bioling water, the specimens were placed in 5% hydrogen peroxide for about 2 hours to complete the removal of residual tissues. Casts were then rinsed in running water overnight.

RNA isolation, RT-PCR and protein anlysis: Total cellular RNA was isolated using RNeasy Mini kit (Qiagen) according to manufacturer's instructions. RNA concentrations and purities were determined spectrophotometrically (Ultrospec 3000, Amersham) as well as by agarose gel electrophoresis followed by ethidium bromide staining. RT-PCRs were performed according to standard protocols by using 75 ng of reverse-transcribed total RNA in a total volume of 50 µl. Real-time RT-PCR analysis of sestrin 2 gene expression in ES cells was performed using SYBR green chemistry (ABgene, Epsom, UK) and an iCycler (Biorad) machine. cDNA was synthesized from total RNA using random priming and Superscript II (Invitrogen) reverse transcriptase. PCR reactions were run as triplicates in 25-µl volumes on 96-well plates, with each reaction containing cDNA derived from 15 ng of total RNA, 1x ABsolute SYBR fluorescein mix (ABGene) and 5 pmol of each of the following primers: 5'-CCTGGAACGGAACCTCAAAATC-3' (SEQ ID NO:8) and 5'-GGGCTTCAAGGAG-CAGCAAG-3'(SEQ ID NO:9). Amplification reactions were allowed to proceed for 35 cycles at 94 °C for 15 s, 61 °C for 30 s, and 72 °C for 30 s. For Western blotting lysates of lung and colon fibroblasts were resolved by SDS-PAGE, transfered to nitrocellulose membranes and reacted with rabbit anti-sestrin 2 polyclonal antibody (PTG Inc., Chicago, IL).

Determination of intracellular peroxide levels: 3 x 10⁵ lung or colon fibroblasts from wild type and *sesn2*^{-/-} mice plated into 6 cm Petri dishes a day before were trypsinized, washed in serum free DMEM and resuspended in 5 ml of serum free DMEM. To some cultures H₂O₂ was added to a final concentration of 200 µM. After incubating for 3 hours dichlorodihydrofluorescein (DCF) was added to the cells at a final concentration of 30 µM and incubated for another hour. After that cell were wahed in PBS, trypsinized and resuspended in 300 µl PBS which were subjected to FACS analysis.

### Example 1: A pT1βgeo gene trap insertion in the sestrin 2 gene induces a null mutation in transgenic mice.

The W077E06 gene trap ES cell line was obtained by electroporating the pT1βgeo gene trap vector shown in SEQ ID NO:10 into TBV-2 (129SvPas) ES cells as previously described (Floss, T. & Wurst, W. Methods Mol Biol 185, 347-79, 2002). Gene trap expressing ES-cell clones were selected in 200 µg/ml G418 (Gibco/BRL), manually picked, expanded, and stored frozen in liquid nitrogen. The gene trap insertion in the 9^{th} intron of the sestrin 2 (*sesn2*) gene was identified among the recovered clones by 5' RACE as described in the Methods section and by sequence database analysis (Genbank at http://www.ncbi.nlm.nih.gov) using the BlastN algorithm. The gene trap insertion in the W077E06 cell line was verified by genomic PCR (see Methods) using the following primers complementary to gene trap- and adjacent upstream intron sequences: 5'-CAGCCTTGAGCCTCTGGAGC-3' (SEQ ID NO:11) and 5'-CTACCCTGAGAAGACGACCCG-3' (SEQ ID NO:12). The veryfied W077E06 ES cells were then converted into mice by blastocyst injection. F1 mice carrying the gene trap allele were intercrossed and the F2 offspring was mated to *ltbp4*^{-/-} mice (WO 03/015505 A3) to obtain double knock-out mice.

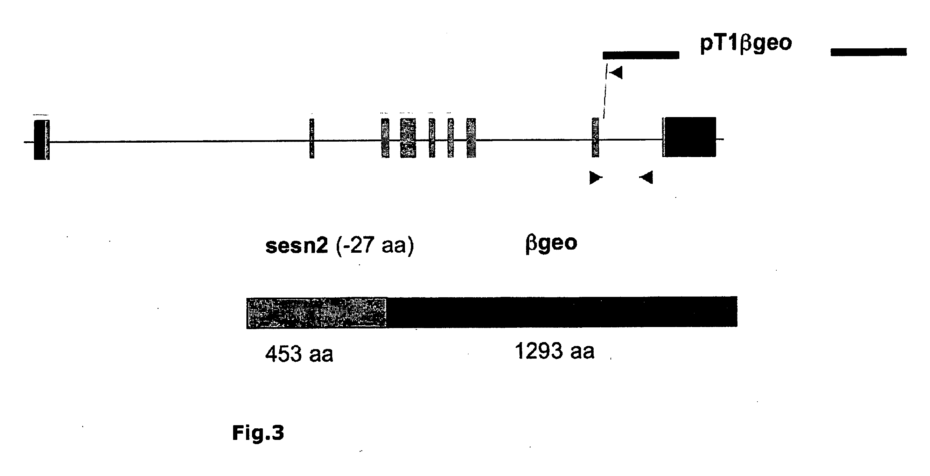

Figure 3 shows the genotyping strategy for the W077E06 (sesn2) mice. Tail DNA was PCR amplified in parallel reactions using a forward primer in the 9^{th} exon (5'-CTACCCTGAGAAGACGACCCG-3'; SEQ ID NO:13) and two reverse primers; one in the 9^{th} intron for detecting the wild type allele (5'-GGACAAATCAAGGTTACACAGAAAAAAGTC-3'; SEQ ID NO:14) and the other in the gene trap's splice acceptor site for detecting the gene trap allele (5'-CAGCCTTGAGCCTCTGGAGC-3'; SEQ ID NO:15). Amplification reactions were allowed to proceed for 30 cycles at 94 °C for 15 s, 61 °C for 30 s, and 72 °C for 30 s.

Intercrossing F1 heterozygous offspring yielded homozygous offspring at a frequency consistent with a Mendelian inheritance pattern of the disrupted transgene, indicating that sesn2 is not required for development. Mutant mice developed normally after birth and were grossly indistinguishable from their wild type and heterozygous littermates.

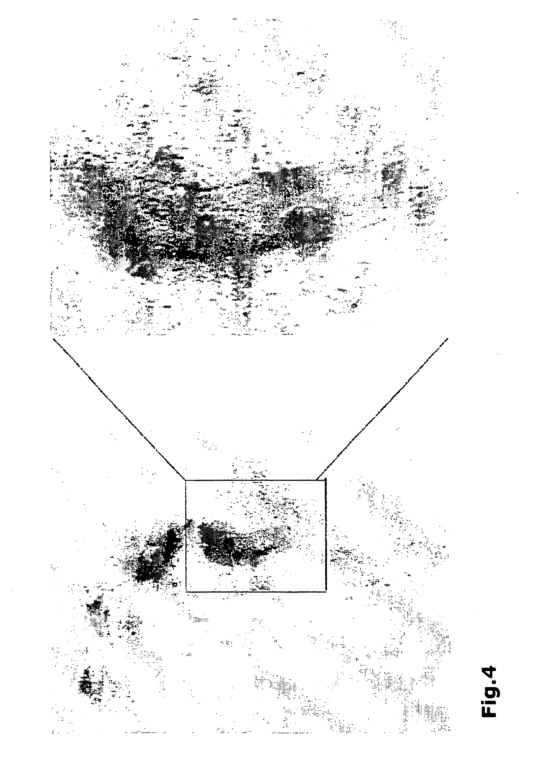

The gene trap insertion in the 9^{th} intron of the *sesn2* gene induces a fusion transcript in which the exons upstream of the insertion are spliced in frame to βgeo. Because transcription is terminated at the gene trap's polyadenylation site, the processed fusion transcript encodes a truncated version of sesn2 and the βgeo reporter (Figure 3). Figure 4 shows faint expression of this protein in an early heterozygous embryo stained with X-Gal.

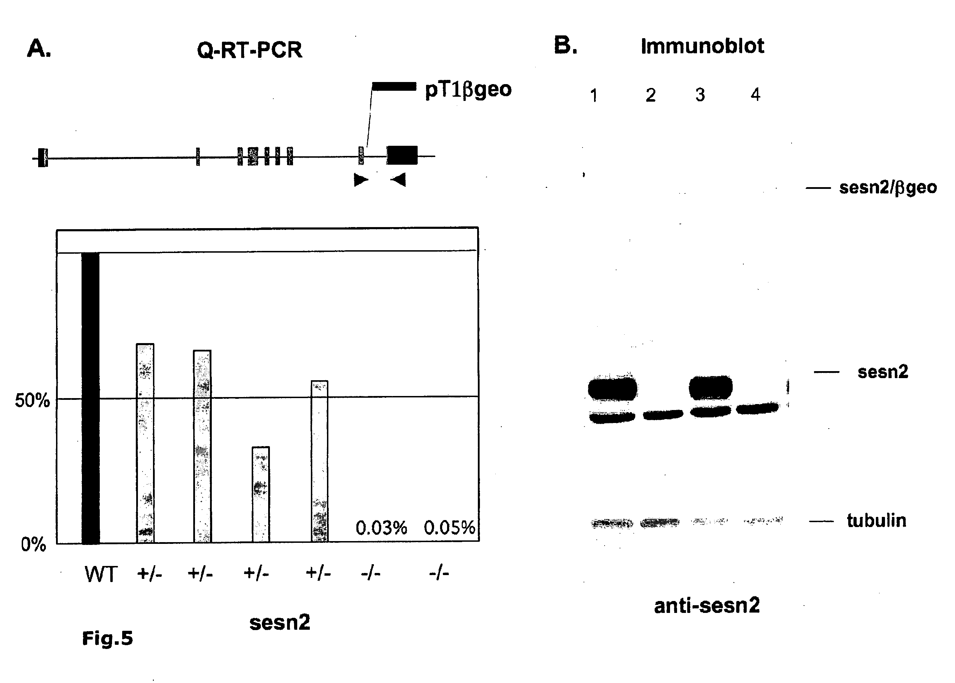

Wild type *sesn2* transcripts were completely lost from lungs of homozygous W077E06 mice whereas heterozygous lungs expressed about 50% of wild type levels (Figure 5A). Moreover, in isolated lung (MLF) and colon (CLF) fibroblasts, which both express high levels of sesn2 (Figure 5B), no protein was detected in cells from homozygous W077E06 mice, suggesting that the gene trap insertion had induced a null mutation (Figure 5B). Consistent with the faint β-gatactosidase expression in the early embryos (Figure 4), mutant MLFs expressed only trace amounts of the sesn2/βgeo fusion protein, implying that the relatively large protein is unstable (Figure 5B). Given its low expression, this protein is unlikely to have a dominant negative effect even if the truncated protein retained some residual function.

### Example 2: sesn2 null alleles improve the pulmonary emphysema in ltbp4^{-/-} mice.

129/Sv (D3) ES cells with a U3Cre gene trap vector (SEQ ID NO:16) insertion in the 5^{th} intron of the ltbp4 gene were injected C57BL/6 blastocysts as previously described (WO 03/015505, Thorey, I.S. et al. Mol Cell Biol 18, 3081-3088, 1998). The resulting male chimeras were bred to C57BL/6 females and agouti offspring were tested for transgene transmission by tail blotting. Mouse tail DNA was cleaved with BgIII which does not cut provirus. The DNA was fractionated on 1% agarose gels, blotted onto Hybond N nylon filters (Amersham/ Pharmacia, Piscataway, NJ) and hybridized to a ³²P labeled provirus flanking sequence probe. Animals heterozygous for the gene trap insertion were backcrossed to C57BL/6 mice for at least six generations before analyzing the phenotypes in heterozygous and homozygous offspring. To obtain double mutant strains, heterozygous *ltbp4*^{*+*/}*⁻* mice were crossed to homozygous *sesn2*^{*-*/}*⁻* mice and the offspring was genotyped by tail DNA PCR as described in Example 1 using the following allele specific primers: ltbp4 wild type allele = 5'-CCAATCTTGCTTCTTTGCTGAGC-3' (SEQ ID NO:17) and 5'-GGC-TCATGCTTGAATGTTTCAG-3' (SEQ ID NO:18); ltbp4 gene trap (mutant allele) = 5'-CCAATCTTGCTTCTTTGCTGAGC-3' (SEQ ID NO:19) and 5'-ATCATGCAAGCTGGTGG-CTG-3' (SEQ ID NO:20); sesn2 wild type allele = 5'-CTACCCTGAGAAGACGACCCG-3' (SEQ ID NO:21) and 5'-GGACAAATCAAGGTTACACAGAAAAAAGTC-3' (SEQ ID NO:22); sesn2 gene trap (mutant allele) = 5'-CTACCCTGAGAAGACGACCCG-3' (SEQ ID NO:23) and 5'-CAGCCTTGAGCCTCTGGAGC-3' (SEQ ID NO:24).

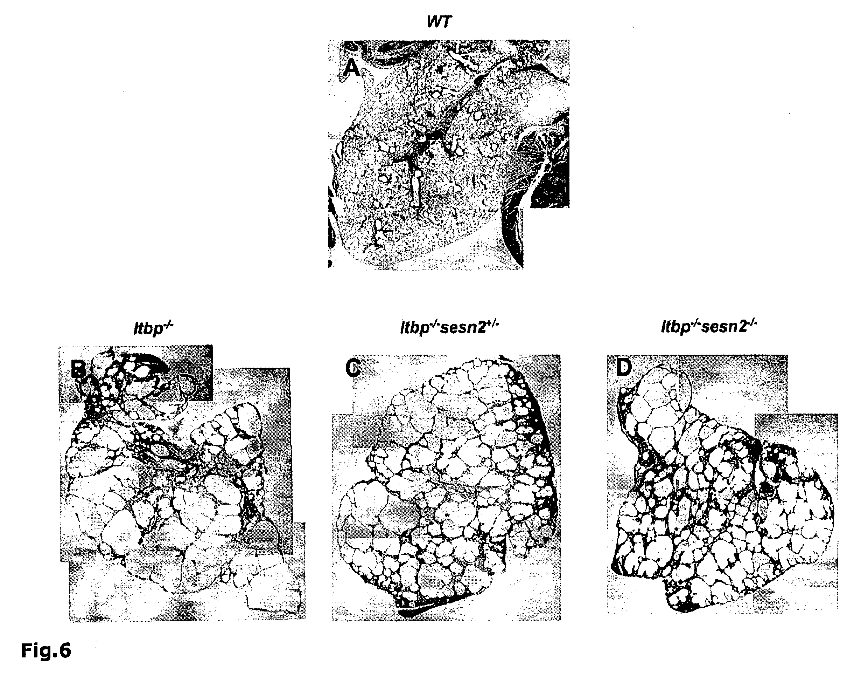

Figure 6B shows a typical emphysematous lung of an adult *ltbp4*^{-/-} mouse (WO 03/015505). It displays massively dilated alveoli surrounded by thin, dysplastic and frequently disrupted septal walls. The lobular connective tissue is significantly reduced, and lungs display multifocal atelectatic areas. Consistent with a partial recovery alveolar spaces in double mutant *ltbp4*^{-/-}*sesn*^{-/-} littermates were more numerous, reduced in size and separated by thicker walls (Figure 6D).

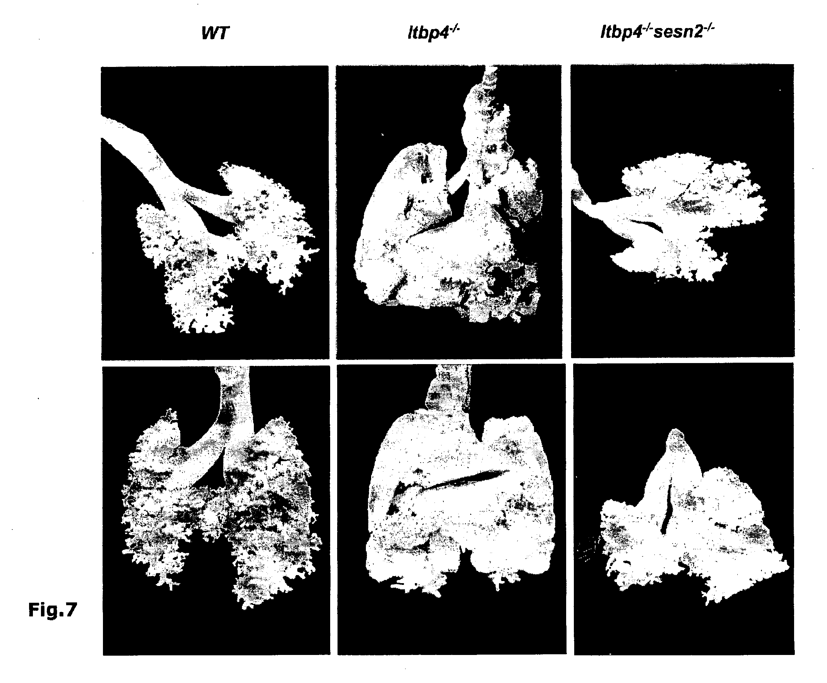

To detect the extent of damage to the bronchioles, alveolar ducts and alveoli, we visualized the tracheobronchial trees of these animals by plastination. Plastination involves the instillation of silicone into the trachea of the isolated lung. Since the alveolar ducts prevent the silicone from entering the alveolar sacs, the technique provides an informative, three dimensional image of the tracheobronchial tree (Perry S. F., et al. Exp Lung Res. 26:27-39, 2000). Figure 7 (left panels) shows the tracheobronchial tree of two 5.5 months old wild type mice with ramifications down to the terminal bronchioles and alveolar ducts. In mutant *ltbp4* littermates, these ramifications were almost completely obscured by enlarged airspaces filled with silicone, suggesting that the terminal bronchioles and alveolar ducts are enlarged and leaky (Figure 7, middle panels). However, a dramatic improvement in the tracheobronchial architecture was observed in *ltbp4*^{*-*/}*⁻* mice carrying two sesn2 null alleles (*ltbp4*^{*-*/}*⁻sesn2*^{*-*/*-*}) (Figure 7, right panels). In contrast to an almost invisible tracheobronchial architecture in *ltbp4*^{*-*/}*⁻* mice, the double mutant tracheobronchial tree was close to normal again, implying a regeneration of terminal bronchioles and alveolar ducts. These modification were much more dramatic than the parenchymal changes presumably reflecting an uneven regeneration of collagen and elastin fibers, which are the major components of the pulmonary ECM (Suki B., et al. J Appl Physiol. 98:1892-9, 2005). Although both form dense fiber networks throughout the lung, the elastin fibers are distributed evenly whereas the collagen fibers tend to condense around terminal bronchioles and alveolar ducts (Toshima M., et al. Arch Histol Cytol. 67:31-40, 2004). Based on this, we speculated that the preferential recovery of the tracheobronchial tree in the *ltbp4*^{*-*/}*⁻sesn2*^{*-*/}*⁻* mice could be the result of an excess collagen deposition into the pulmonary ECM.

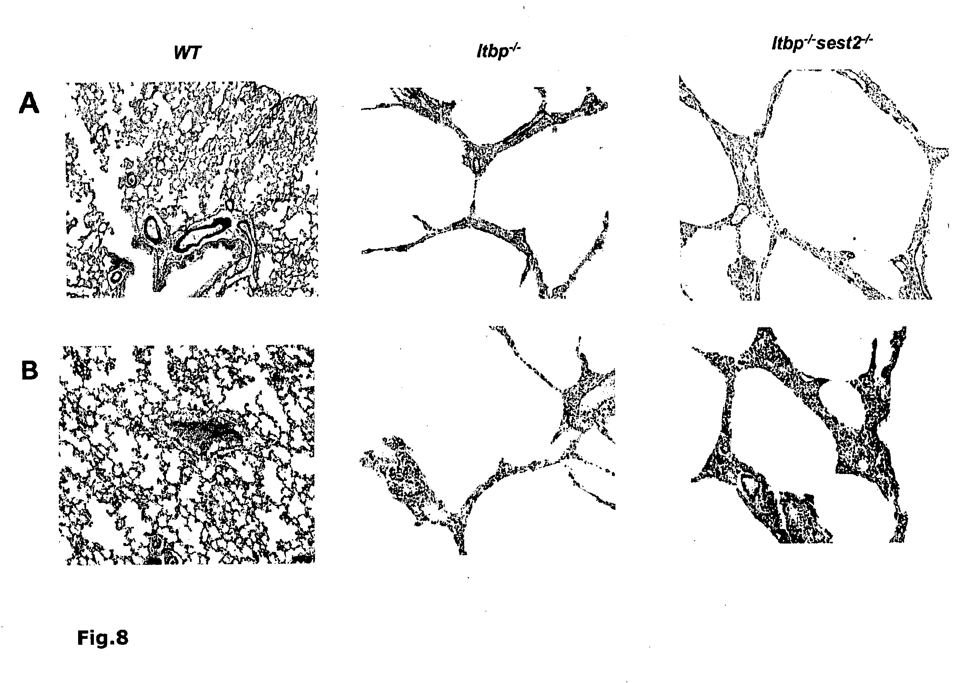

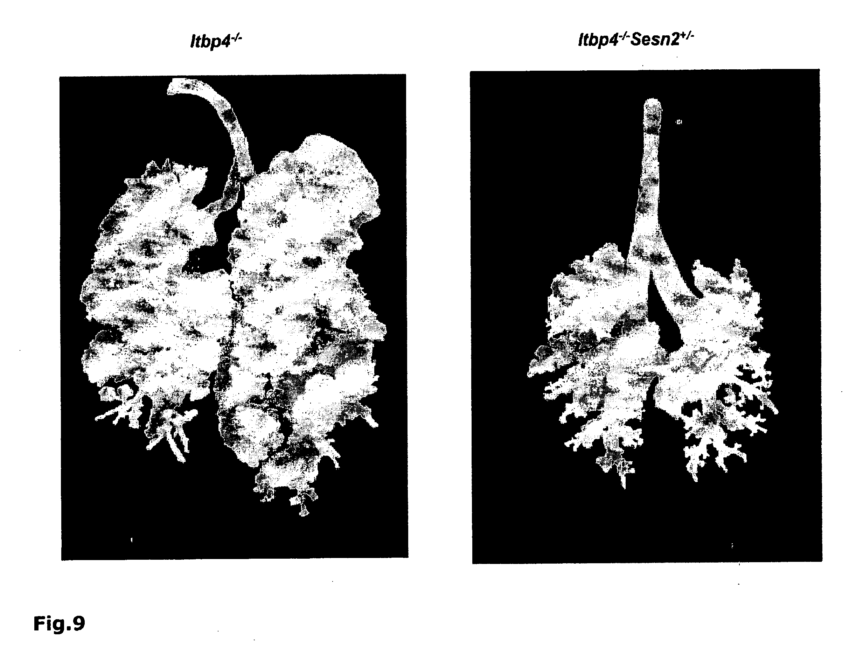

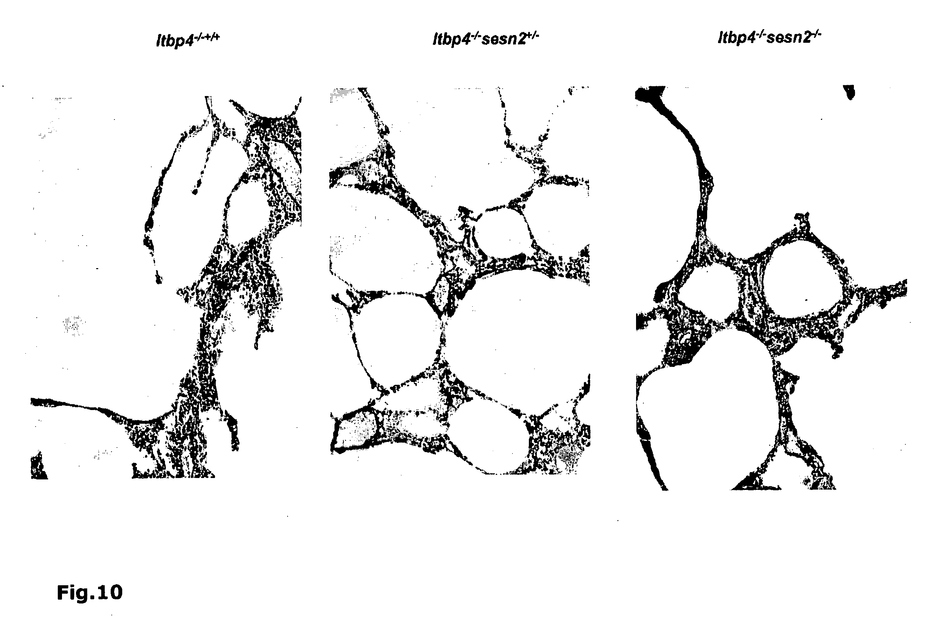

To test this, we visualized elastin and collagen in lung tissue sections using specific histochemical stains. Figure 8A shows the elastin network of *ltbp4*^{*-*/}*⁻* and *ltbp4*^{*-*/}*⁻sesn2*^{*-*/}*⁻* lungs appearing fragmented, patchy and condensed in both. In contrast, collagen deposition was dramatically increased in the double mutant lungs (Figure 8B). As TGF-β is one of the most potent collagen inducers, the excessive deposition of collagen suggested a reactivation of TGF-β signaling in the *ltbp4*^{*-*/}*⁻sesn2*^{*-*/}*⁻* lungs. Since trancheobronchial recovery and increased deposition of collagen was also noticed in *ltbp4*^{*-*/}*⁻* mice heterozygous for sesn2 (*ltbp4*^{*-*/}*⁻sesn2*^{*+*/*-*}) (Figures 9, 10), the *sesn2* mutation could be haploinsufficient.

### Example 3: sesn2 null alleles improve the rectal prolapse and colorectal adenomas in ltbp4^{-/}⁻ mice.

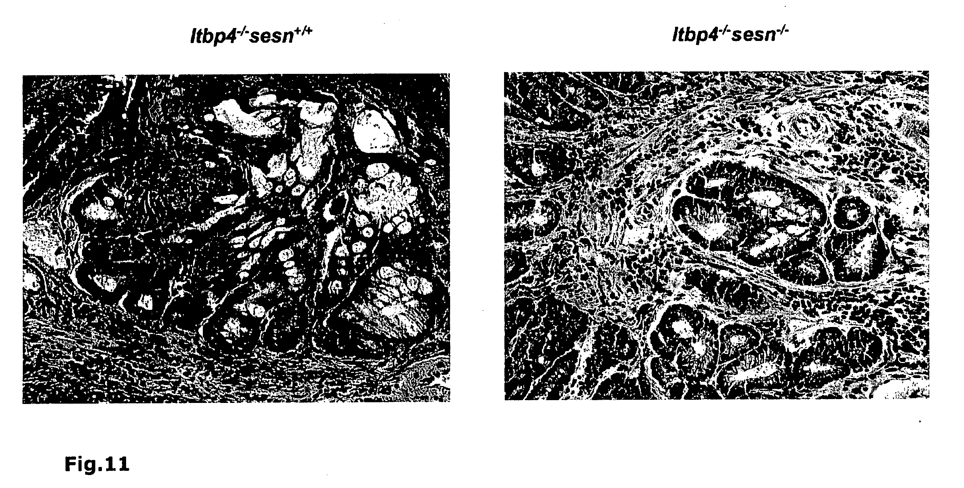

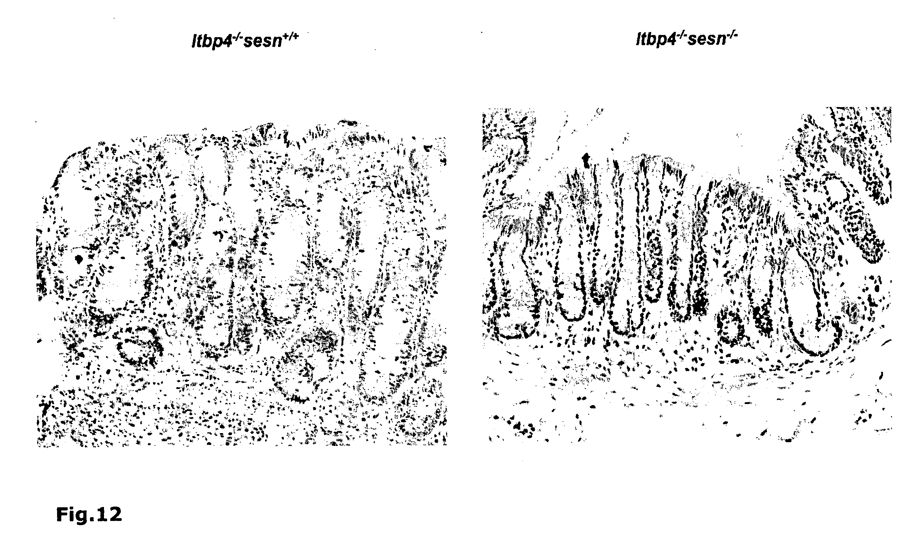

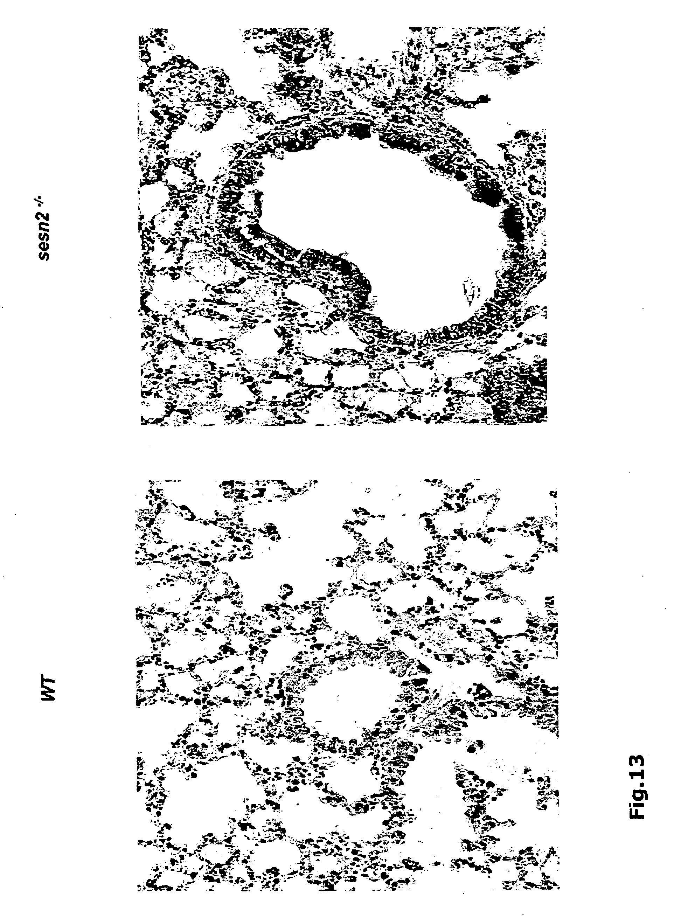

Figure 13 shows a typical colorectal an adenoma of 3 months old *ltbp4*^{*-*/}*⁻* (WO 03/015505 A3) mouse. Microscopically, the region exhibited abberant crypt foci containing regenerating epithelial cells and an increased number of goblet cells. Although double mutant *ltbp4*^{*-*/}*⁻sesn^{-l-}* littermates also exhibited adenomas, these were sigificantly smaller in size and contained fewer goblet cells (Figure 11), suggesting a partial phenotypic rescue. Since the ltbp4-/- phenotype is essentially caused by defective TGF-β activation (Sterner-Kock A., et al. Genes Dev. 16:2264-2273, 2002), we tested wether the partial rescue was associated with a reactivation of TGF-β signaling. Towards this end we determined the levels of phosphorylated smad2 in tissue sections by immunohistochemistry. Consistent with an activation of TGF-β signaling in *ltbp4*^{*-*/}*⁻sesn2*^{*-*/}*⁻* mice, p-smad2 levels werevery high in contrast to the single mutant *ltbp4*^{*-*/}*⁻* mice, which have no detectable P-smad2 in their colon (Figure 12).

### Example 4: Sesn2^{-/}⁻ lungs exhibit enhanced TGF-β signaling.

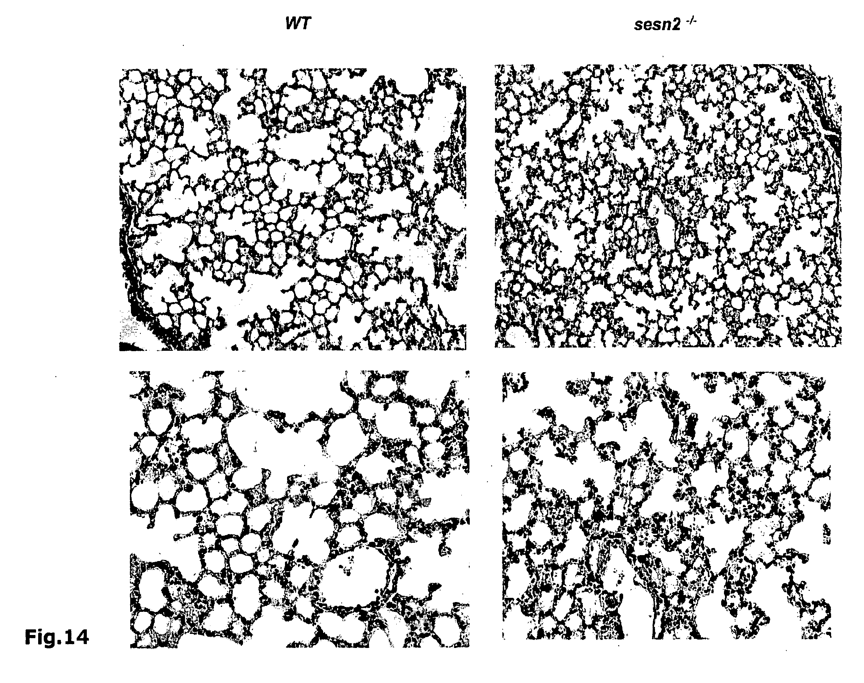

The increased collagen deposition in the lung and the increased P-smad2 levels in the colon of *ltbp4*^{*-*/}*⁻sesn2*^{*-*/}*⁻* mice suggested the TGF-β pathway might be similarly activated in the *sesn2*^{*-*/}*⁻* mice despite their grossly normal phenotype. To test this we visualized P-smad2 in lung tissue sections as described above. In a preliminary experiment, we found that P-smad2 levels in *sesn^{-l-}* lungs significantly exceeded the wild type levels, suggesting that loss of sestrin 2 activates TGF-β (Figure 13). Moreover, although we could not detect increased collagen deposition by Mason's trichrome stain (data not shown), the *sesn2*^{*-*/}*⁻* lungs exhibited more abundant connective tissue, smaller airspaces and thicker interalveolar walls, which are all consistent with incipient, TGF-β induced fibrosis (Figure 14) Sime P. J., et al. J Clin Invest. 100:768-76, 1997; Lee M. S., et al. Am J Pathol. 147:42-52, 1995; Sanderson N., et al. Proc Natl Acad Sci U S A. 92:2572-6, 1995).

### Example 5: Increased peroxide accumulation in mouse lung fibroblasts (MLFs) derived from sesn^{-l-} mice.

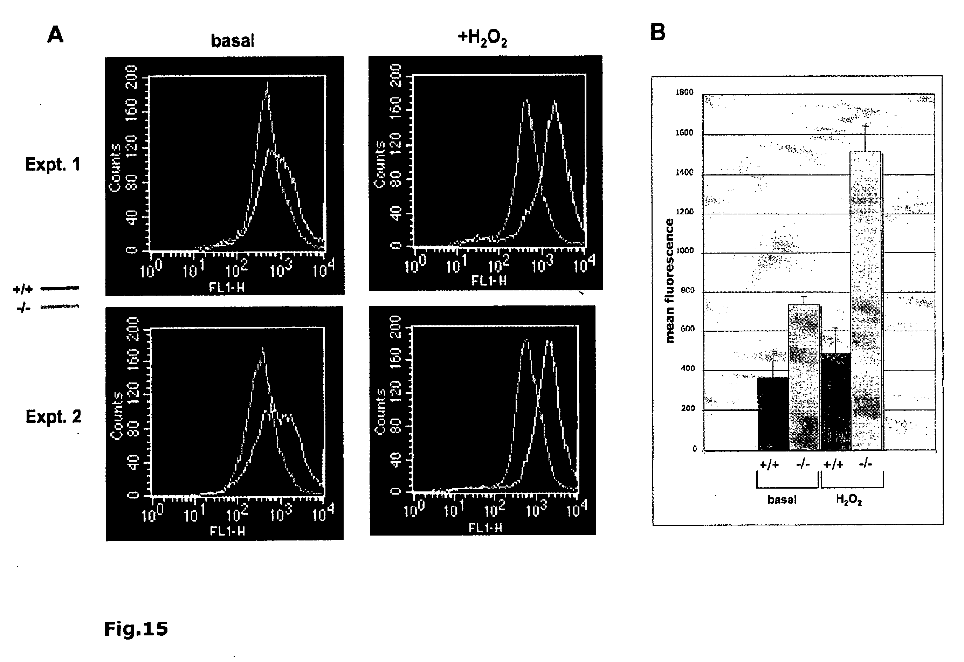

Recently published experiments in have shown that the inhibition of sesn2 expression by shRNA compromises the cell's ability to process ROS leading to ROS accumulation and oxidative stress (Budanov A. V., et al. Science. 304:596-600, 2004). In line with this, we expected antioxidant functions to be similarly compromised in cells derived from sesn2 knock out mice. To test this, we quantified basal and H₂O₂ induced ROS levels in MLFs using the dichlorodihydrofluorescein (DCF) fluorescence method. Figure 15 shows that *sesn^{-l-}* MLFs accumulated significantly more ROS than the corresponding wild type cells, regardless of pretreatment with 200 mM H₂O₂. The results are equivalent to those obtained in the previously described shRNA knock down experiments and are consistent with a sesn2 loss of function in the mutant MLFs.

**Sequence Listing, Free Text**

| | | |
|---|---|---|
| SEQ ID NOs:1-9 | Primer | |
| SEQ ID NO:10 | Gene trap vector pTβgeo plasmid Elements: | |
| | - En-2 Splice Acceptor | 2284-4163 |
| | βGeo | 4164-8053 |
| | - SV40 pA | 8054-8496 |
| | - plasmid backbone | 8497-2283 |
| SEQ ID NOs:11-15 | Primer | |
| SEQ ID NO:16 | Gene trap vector U3Cre Plasmid elements: | |
| | - LTRs 1-1475 and 3046-4520 | |
| | - Cre 3-1090 and 3074-4133 | |
| | - gag/env: 1475 - 3045. | |
| SEQ ID NOs:17-24 | Primer | |

## Claims

1. A rodent animal model, which does not express one or more functional sestrins or expresses translational levels reduced at least by 50% of one or more sestrins and does not express latent transforming growth factor β binding protein 4 (ltbp4) or expresses translational levels reduced at least by 50% of ltbp4.

2. The rodent animal model of claim 1, wherein
(i) the genome of the rodent comprises a homozygous or a heterozygous disruption of a sestrin gene, preferably the disrupted sestrin gene being selected from sestrin 1, sestrin 2, and sestrin 3, and most preferably being sestrin 2; and/or
(ii) the genome of the rodent comprises a homozygous or a heterozygous disruption of the ltbp4 gene.

3. The rodent animal model of claim 2, wherein the disruption being generated by a mutation, preferably said mutation
(i) is an insertion, deletion or substitution mutation; and/or
(ii) is generated by gene targeting, gene trapping or chemical mutagenesis; and/or
(iii) has occurred in an exon, intron, regulatory region or splice site of the sestrin and ltbp4 genes; and/or
(iv) gives rise to the expression of a reporter gene; and/or
(v) has occurred in the 9^{th} intron of the sestrin 2 gene; and/or
(vi) has occurred in the 5^{th} intron of the ltpb4 gene.

4. The rodent animal model of claim 2 or 3, wherein
(i) the sestrin 2 gene is disrupted in the 9^{th} intron by inserting a gene trap vector, preferably pT1bgeo (SEQ ID NO:10); and/or
(ii) the ltbp4 gene is disrupted in the 5^{th} intron by inserting a gene trap vector, preferably U3Cre (SEQ ID NO:16).

5. The rodent animal model of any one of claims 1 to 4, wherein
(i) said rodent is a mouse or rat; and/or
(ii) said rodent exhibits pulmonary emphysema and cancer, preferably the cancer is a colon cancer.

6. A rodent animal model which does not express one or more functional sestrins or expresses translational levels reduced at least by 50% of one or more sestrins, preferably said rodent animal model being as defined in claims 1 to 5.

7. A cell or tissue culture isolated from the rodent animal models of claims 1 to 6, wherein levels of expression, in the cell or in the cells of the tissue, of the sestrin and ltbp4 proteins are as defined in claims 1 to 6.

8. The cell or tissue culture of claim 7, wherein the cell or tissue culture is from lung or colon.

9. A method for preparing the rodent animal model of claims 1 to 6, which comprises disrupting the sestrin and/or ltbp4 gene in a germ cell of a starting rodent.

10. The method of claim 9, wherein the germ cell is an ES cell.

11. A method for selecting an agent for treating a symptom occurring in the rodent animal model of claim 1 comprising:
(i) applying one or more agents to be tested to said rodent animal model,
(ii) determining whether one or more symptoms occurring in said rodent animal model have changed as a result of application of said agent(s).

12. The method of claim 11, wherein the symptom is selected from a group consisting of cancer and pulmonary emphysema.

13. A method of selecting an agent that interferes with ROS production and TGF-β signaling comprising:
(i) applying one or more agents to be tested to the to the cell or tissue culture of claim 7,
(ii) determining whether cellular ROS levels and TGF-β signaling have changed as a result of the application of said agents or agents.

14. The method of claim 13, wherein the cell or tissue culture is from lung or colon.

15. A method to analyze whether cancer and/or pulmonary emphysema is caused by differential ltbp4 and sestrin gene or protein expression or expression level or by a defect in the ltbp4 and sestrin gene comprising:
(i) characterizing the ltbp4 and sestrin gene or protein expression or expression level or ltbp4 and sestrin gene allele status of an individual having cancer or pulmonary emphysema,
(ii) characterizing the ltbp4 and sestrin gene or protein expression or expression level or ltbp4 and sestrin gene allele status of a control individual, a difference in the ltbp4 and sestrin gene or protein expression or expression level or ltbp4 and sestrin gene allele status indicating that cancer and/or pulmonary emphysema and/or cardiomyopathy is linked to differential ltbp4 and sestrin gene or protein expression or expression level or a defect in the ltbp4 and sestrin genes.

16. The method of claim 15, wherein
(i) the ltbp4 and sestrin expression or expression level is detected by RT-PCR, Northern analysis, microarray analysis or antibodies directed to ltbp4 and sestrin proteins; and/or
(ii) the ltbp4 and sestrin gene expression or expression level is detected by RT-PCR, Northern analysis, microarray analysis or antibodies directed to ltbp4 and sestrin proteins; and/or
(iii) the ltbp4 and sestrin gene allele status is detected by mutation screening.

## Patentansprüche

1. Nagetiermodell, das ein oder mehrere funktionelle Sestrine nicht exprimiert oder um wenigstens 50% reduzierte Translationsmengen eines oder mehrerer Sestrine exprimiert und latenten transformierenden Wachstumsfaktor β bindendes Protein 4 (ltbp4) nicht exprimiert oder um wenigstens 50% reduzierte Translationsmengen von ltbp4 exprimiert.

2. Nagetiermodell gemäß Anspruch 1, wobei
(i) das Genom des Nagetiers eine homozygote oder heterozygote Disruption eines Sestrin-Gens umfasst, wobei das disrumpierte Sestrin-Gen vorzugsweise aus Sestrin 1, Sestrin 2 und Sestrin 3 ausgewählt ist und es sich am meisten bevorzugt um Sestrin 2 handelt; und/oder
(ii) das Genom des Nagetiers eine homozygote oder heterozygote Disruption des ltbp4-Gens umfasst.

3. Nagetiermodell gemäß Anspruch 2, wobei die Disruption durch eine Mutation erzeugt wird, wobei die Mutation vorzugsweise
(i) eine Insertions-, Deletions- oder Substitutionsmutation ist; und/oder
(ii) durch gezielte Genmodifikation (targeting), Gene Trapping oder chemische Mutagenese erzeugt ist; und/oder
(iii) in einem Exon, Intron, regulatorischen Bereich oder einer Spleißstelle des Sestrin- und ltbp4-Gens aufgetreten ist; und/oder
(iv) zur Expression eines Reporter-Gens führt; und/oder
(v) im neunten Intron des Sestrin-2-Gens aufgetreten ist; und/oder
(vi) im fünften Intron des ltbp4-Gens aufgetreten ist.

4. Nagetiermodell gemäß Anspruch 2 oder 3, wobei
(i) das Sestrin-2-Gen im neunten Intron durch Inserieren eines Genfallen-Vektors, vorzugsweise pT1bgeo (SEQ ID Nr. 10) disrumpiert ist; und/oder
(ii) das ltbp4-Gen im fünften Intron durch Inserieren eines Genfallen-Vektors, vorzugsweise U3Cre (SEQ ID Nr. 16) disrumpiert ist.

5. Nagetiermodell gemäß einem der Ansprüche 1 bis 4, wobei
(i) das Nagetier eine Maus oder Ratte ist; und/oder
(ii) das Nagetier ein Lungenemphysem und Krebs aufweist, wobei der Krebs vorzugsweise ein Kolonkarzinom ist.

6. Nagetiermodell, das ein oder mehrere funktionelle Sestrine nicht exprimiert oder um wenigstens 50% reduzierte Translationsmengen eines oder mehrerer Sestrine exprimiert, wobei das Nagetiermodell vorzugsweise wie in den Ansprüchen 1 bis 5 definiert ist.

7. Zell- oder Gewebekultur, isoliert aus den Nagetiermodellen gemäß Anspruch 1 bis 6, wobei die Expressionsmengen der Sestrin- und ltbp4-Proteine in der Zelle oder in den Zellen des Gewebes wie in den Ansprüchen 1 bis 6 definiert sind.

8. Zell- oder Gewebekultur gemäß Anspruch 7, wobei die Zell- oder Gewebekultur aus Lunge oder Kolon stammt.

9. Verfahren zur Herstellung des Nagetiermodells gemäß Anspruch 1 bis 6, umfassend das Disrumpieren des Sestrin- und/oder ltbp4-Gens in einer Keimzelle eines Ausgangsnagetiers.

10. Verfahren gemäß Anspruch 9, wobei die Keimzelle eine ES-Zelle ist.

11. Verfahren zum Selektieren eines Mittels zur Behandlung eines Symptoms, das im Nagetiermodell gemäß Anspruch 1 auftritt, umfassend:
(i) Anwenden eines oder mehrerer zu testender Mittel auf das Nagetiermodell;
(ii) Bestimmen, ob sich ein oder mehrere Symptome, die in dem Nagetiermodell auftreten, infolge der Anwendung des bzw. der Mittel verändert haben.

12. Verfahren gemäß Anspruch 11, wobei das Symptom aus der Gruppe ausgewählt ist, die aus Krebs und Lungenemphysem besteht.

13. Verfahren zum Selektieren eines Mittels, das die ROS-Produktion und den TGF-β-Signalweg stört, umfassend:
(i) Anwenden eines oder mehrerer zu testender Mittel auf die Zell- oder Gewebekultur gemäß Anspruch 7;
(ii) Bestimmen, ob sich die zellulären ROS-Konzentrationen und der TGF-β-Signalweg infolge der Anwendung des bzw. der Mittel verändert haben.

14. Verfahren gemäß Anspruch 13, wobei die Zell- oder Gewebekultur aus Lunge oder Kolon stammt.

15. Verfahren zum Analysieren, ob Krebs und/oder Lungenemphysem durch differentielle ltbp4- und Sestrin-Gen- oder -Proteinexpression oder -expressionsmenge oder durch einen Defekt im ltbp4- und Sestrin-Gen verursacht ist, umfassend:
(i) Charakterisieren der ltbp4- und Sestrin-Gen- oder -Proteinexpression oder -expressionsmenge oder des ltbp4- und Sestrin-Gen-Allelstatus eines Individuums, das an Krebs oder Lungenemphysem leidet;
(ii) Charakterisieren der ltbp4- und Sestrin-Gen- oder -Proteinexpression oder -expressionsmenge oder des ltbp4- und Sestrin-Gen-Allelstatus eines Kontrollindividuums, wobei ein Unterschied in der ltbp4- und Sestrin-Gen-oder -Proteinexpression oder -expressionsmenge oder des ltbp4- und Sestrin-Gen-Allelstatus anzeigt, dass Krebs und/oder Lungenemphysem und/oder Kardiomyopathie mit differentieller ltbp4- und Sestrin-Gen- oder -Proteinexpression oder -expressionsmenge oder einem Defekt im ltbp4-und Sestrin-Gen zusammenhängt.

16. Verfahren gemäß Anspruch 15, wobei
(i) die ltbp4- und Sestrin-Expression oder -Expressionsmenge durch RT-PCR, Northern-Analyse, Mikroarray-Analyse oder gegen ltbp4- und Sestrin-Protein gerichtete Antikörper nachgewiesen wird; und/oder
(ii) die ltbp4- und Sestrin-Genexpression oder -expressionsmenge durch RT-PCR, Northern-Analyse, Mikroarray-Analyse oder gegen ltbp4- und Sestrin-Protein gerichtete Antikörper nachgewiesen wird; und/oder
(iii) der ltbp4- und Sestrin-Gen-Allelstatus durch Mutationsscreening nachgewiesen wird.

## Revendications

1. Modèle animal de rongeur qui n'exprime pas une ou plusieurs sestrines fonctionnelles ou exprime des niveaux traductionnels réduits d'au moins 50% d'une ou de plusieurs sestrines et qui n'exprime pas la protéine de liaison du facteur de croissance transformant β lat ente 4 (ltbp4) ou exprime des niveaux traductionnels réduits d'au moins 50% de la ltbp4.

2. Modèle animal de rongeur selon la revendication 1, dans lequel
(i) le génome du rongeur comprend une disruption homozygote ou hétérozygote d'un gène de sestrine, de préférence, le gène de sestrine présentant une disruption étant choisi parmi la sestrine 1, la sestrine 2 et la sestrine 3, et le plus préférablement, la sestrine 2; et/ou
(ii) le génome du rongeur comprend une disruption homozygote ou hétérozygote du gène de ltbp4.

3. Modèle animal de rongeur selon la revendication 2, dans lequel la disruption est générée par une mutation, de préférence, ladite mutation
(i) est une mutation par insertion, délétion ou substitution; et/ou
(ii) est générée par un ciblage de gènes, un piégeage de gènes ou une mutagenèse chimique; et/ou
(iii) a eu lieu dans un exon, un intron, une région régulatrice ou un site d'épissage des gènes de sestrine et de ltbp4; et/ou
(iv) donne lieu à l'expression d'un gène rapporteur; et/ou
(v) a lieu dans le 9^{ème} intron du gène de la sestrine2; et/ou
(vi) a lieu dans le 5^{ème} intron du gène de la ltbp4.

4. Modèle animal de rongeur selon la revendication 2 ou 3, dans lequel
(i) le gène de la sestrine 2 est interrompu dans le 9^{ème} intron par insertion d'un vecteur de piégeage de gènes, de préférence, pTlbgeo (SEQ ID NO:10); et/ou
(ii) le gène de ltbp4 est interrompu dans le 5^{ème} intron par insertion d'un vecteur de piégeage de gènes, de préférence, U3Cre (SEQ ID NO:16).

5. Modèle animal de rongeur selon l'une quelconque des revendications 1 à 4, dans lequel
(i) ledit rongeur est une souris ou un rat; et/ou
(ii) ledit rongeur présente un emphysème pulmonaire et un cancer, de préférence, le cancer est un cancer du côlon.

6. Modèle animal de rongeur qui n'exprime pas une ou plusieurs sestrines fonctionnelles ou exprime des niveaux traductionnels réduits d'au moins 50% d'une ou de plusieurs sestrines, de préférence, ledit modèle animal de rongeur étant tel que défini dans les revendications 1 à 5.

7. Culture cellulaire ou tissulaire isolée à partir des modèles animaux de rongeurs selon les revendications 1 à 6, dans laquelle les niveaux d'expression, dans la cellule ou dans les cellules du tissu, des protéines sestrine et ltbp4 sont tels que définis dans les revendications 1 à 6.

8. Culture cellulaire ou tissulaire selon la revendication 7, où ladite culture cellulaire ou tissulaire provient du poumon ou du côlon.

9. Procédé de préparation du modèle animal de rongeur selon les revendications 1 à 6, qui comprend la disruption du gène de sestrine et/ou de ltbp4 dans une cellule germinale d'un rongeur de départ.

10. Procédé selon la revendication 9, dans lequel la cellule germinale est une cellule ES.

11. Procédé de sélection d'un agent servant à traiter un symptôme apparaissant dans le modèle animal de rongeur de la revendication 1, comprenant:
(i) l'administration d'un ou de plusieurs agents à tester audit modèle animal de rongeur,
(ii) la détermination pour savoir si un ou plusieurs symptômes apparus dans ledit modèle animal de rongeur a/ont changé à l'issu de l'administration dudit ou desdits agent(s).

12. Procédé selon la revendication 11, dans lequel le symptôme est choisi dans un groupe constitué par un cancer et un emphysème pulmonaire.

13. Procédé de sélection d'un agent qui interfère avec la production de ROS et la transmission de signal de Tuf-β, comprenant:
(i) l'application d'un ou de plusieurs agents à tester à la culture cellulaire ou tissulaire de la revendication 7,
(ii) la détermination pour savoir si les niveaux cellulaires de ROS et la transmission de signal de TGF-β ont changé à l'issu de l'application dudit ou desdits agents.

14. Procédé selon la revendication 13, dans lequel la culture cellulaire ou tissulaire provient du poumon ou du côlon.

15. Procédé pour analyser si le cancer et/ou l'emphysème pulmonaire est/sont dus à un niveau d'expression ou à une expression différentiel(le) de la protéine ou du gène de ltbp4 et de sestrine ou à une anomalie dans le gène de ltbp4 et de sestrine, comprenant:
(i) la caractérisation du niveau d'expression ou de l'expression de la protéine ou du gène de ltbp4 et de sestrine ou du statut allélique du gène de ltbp4 et de sestrine d'un individu atteint d'un cancer ou d'un emphysème pulmonaire,
(ii) la caractérisation du niveau d'expression ou de l'expression de la protéine ou du gène de ltbp4 et de sestrine ou du statut allélique du gène de ltbp4 et de sestrine d'un individu témoin, d'une différence du niveau d'expression ou de l'expression de la protéine ou du gène de ltbp4 et de sestrine ou du statut allélique du gène de ltbp4 et de sestrine indiquant que le cancer et/ou l'emphysème pulmonaire et/ou la cardiomyopathie est/sont lié(s) à un niveau d'expression ou à une expression différentiel(le) de la protéine ou du gène de ltbp4 et de sestrine ou à une anomalie dans les gènes de ltbp4 et de sestrine.

16. Procédé selon la revendication 15, dans lequel
(i) l'expression ou le niveau d'expression de ltbp4 et de sestrine est détecté par RT-PCR, analyse de Northem, analyse de "microarray" ou des anticorps dirigés contre les protéines ltbp4 et sestrine; et/ou
(ii) l'expression ou le niveau d'expression du gène de ltbp4 et de sestrine est détecté par RT-PCR, analyse de Northem, analyse de "microarray" ou des anticorps dirigés contre les protéines ltbp4 et sestrine; et/ou
(iii) le statut allélique du gène de ltbp4 et de sestrine est détecté par criblage de mutation.


### SEQUENCE LISTING

<110> FrankGen Biotechnologie AG
<120> Animal Model for the Selection and Validation of Agents Active Against Pulmonary Emphysema and Colorectal Cancer
<130> 071673wo/JH
<150> EP 06120714.8
   <151> 2006-09-15
<160> 24
<170> PatentIn version 3.3
<210> 1
   <211> 492
   <212> PRT
   <213> Mus musculus
<400> 1
   Met Arg Leu Ala Ala Ala Ser Asn Glu Ala Tyr Ala Ala Ser Leu Ala
   1               5                   10                  15
   Val Ser Glu Leu Leu Ser Cys His Gln Cys Gly Gly Asp Arg Gly Gln
               20                  25                  30
   Asp Glu Glu Leu Gly Ile Arg Ile Pro Arg Pro Leu Gly His Gly Pro
           35                  40                  45
   Ser Arg Phe Ile Pro Glu Lys Glu Met Leu Gln Val Gly Ser Glu Asp
       50                  55                  60
   Ala Gln Met His Ala Leu Phe Ala Asp Ser Phe Ala Ala Leu Gly Arg
   65                  70                  75                  80
   Leu Asp Asn Ile Thr Leu Val Met Val Phe His Pro Gln Tyr Leu Glu
                   85                  90                  95
   Ser Phe Leu Lys Thr Gln His Tyr Leu Leu Gln Met Asp Gly Pro Leu
               100                 105                 110
   Pro Leu His Tyr Arg His Tyr Ile Gly Ile Met Ala Ala Ala Arg His
           115                 120                 125
   Gln Cys Ser Tyr Leu Val Asn Leu His Val Ser Asp Phe Leu His Val
       130                 135                 140
   Gly Gly Asp Pro Lys Trp Leu Asn Gly Leu Glu Asn Ala Pro Gln Lys
   145                 150                 155                 160
   Leu Gln Asn Leu Gly Glu Leu Asn Lys Val Leu Ala His Arg Pro Trp
                   165                 170                 175
   Leu Ile Thr Lys Glu His Ile Glu Gly Leu Leu Lys Ala Glu Glu His
               180                 185                 190
   Ser Trp Ser Leu Ala Glu Leu Val His Ala Val Val Leu Leu Thr His
           195                 200                 205
   Tyr His Ser Leu Ala Ser Phe Thr Phe Gly Cys Gly Ile Ser Pro Glu
       210                 215                 220
   Ile His Cys Asp Gly Gly His Thr Phe Arg Pro Pro Ser Val Ser Asn
   225                 230                 235                 240
   Tyr Cys Ile Cys Asp Ile Thr Asn Gly Asn His Ser Val Asp Glu Met
                   245                 250                 255
   Gln Val Asn Ser Ala Gly Asn Ala Ser Val Ser Asp Ser Phe Phe Glu
               260                 265                 270
   Val Glu Ala Leu Met Glu Lys Met Arg Gln Leu Gln Glu Cys Arg Glu
           275                 280                 285
   Glu Glu Glu Ala Ser Gln Glu Glu Met Ala Ser Arg Phe Glu Met Glu
       290                 295                 300
   Lys Arg Glu Ser Met Phe Val Phe Ser Ser Asp Asp Asp Glu Val Thr
   305                 310                 315                 320
   Pro Ala Arg Asp Val Ser Arg His Phe Glu Asp Thr Ser Tyr Gly Tyr
                   325                 330                 335
   Lys Asp Phe Ser Arg His Gly Met His Val Pro Thr Phe Arg Val Gln
               340                 345                 350
   Asp Tyr Cys Trp Glu Asp His Gly Tyr Ser Leu Val Asn Arg Leu Tyr
           355                 360                 365
   Pro Asp Val Gly Gln Leu Ile Asp Glu Lys Phe His Ile Pro Tyr Asn
       370                 375                 380
   Leu Thr Tyr Asn Thr Met Ala Met His Lys Asp Val Asp Thr Ser Met
   385                 390                 395                 400
   Leu Arg Arg Ala Ile Trp Asn Tyr Ile His Cys Met Phe Gly Ile Arg
                   405                 410                 415
   Tyr Asp Asp Tyr Asp Tyr Gly Glu Ile Asn Gln Leu Leu Asp Arg Ser
               420                 425                 430
   Phe Lys Val Tyr Ile Lys Thr Val Val Cys Thr Pro Glu Lys Val Thr
           435                 440                 445
   Lys Arg Met Tyr Asp Ser Phe Trp Arg Gln Phe Lys His Ser Glu Lys
       450                 455                 460
   Val His Val Asn Leu Leu Leu Ile Glu Ala Arg Met Gln Ala Glu Leu
   465                 470                 475                 480
   Leu Tyr Ala Leu Arg Ala Ile Thr Arg Tyr Met Thr
                   485                 490
<210> 2
   <211> 492
   <212> PRT
   <213> Mus musculus
<400> 2
   Met Asn Arg Gly Gly Ser Ser Ala Ser Ala Ser Ala Asn Tyr Leu Leu
   1               5                   10                  15
   Cys Thr Asn Cys Arg Lys Val Leu Arg Lys Asp Lys Arg Ile Arg Val
               20                  25                  30
   Ser Gln Pro Leu Thr Arg Gly Pro Ser Ala Phe Ile Pro Glu Lys Glu
           35                  40                  45
   Val Val Gln Ala Asn Thr Ala Asp Glu Arg Thr Asn Phe Leu Val Glu
       50                  55                  60
   Glu Tyr Ser Thr Ser Gly Arg Leu Asp Asn Ile Thr Gln Val Met Ser
   65                  70                  75                  80
   Leu His Thr Gln Tyr Leu Glu Ser Phe Leu Arg Ser Gln Phe Tyr Met
                   85                  90                  95
   Leu Arg Met Asp Gly Pro Leu Pro Leu Pro Asp Arg His Tyr Ile Ala
               100                 105                 110
   Ile Met Ala Ala Ala Arg His Gln Cys Ser Tyr Leu Ile Asn Met His
           115                 120                 125
   Val Asp Glu Phe Leu Lys Thr Gly Gly Ile Ala Glu Trp Leu Asn Gly
       130                 135                 140
   Leu Glu Tyr Val Pro Gln Arg Leu Arg Asn Leu Asn Glu Ile Asn Lys
   145                 150                 155                 160
   Leu Leu Ala His Arg Pro Trp Leu Ile Thr Lys Glu His Ile Gln Lys
                   165                 170                 175
   Leu Val Lys Thr Gly Glu Asn Asn Trp Ser Leu Pro Glu Leu Val His
               180                 185                 190
   Ala Val Val Leu Leu Ala His Tyr His Ala Leu Ala Ser Phe Val Phe
           195                 200                 205
   Gly Ser Gly Ile Asn Pro Glu Arg Asp Pro Gly Ile Ala Asn Gly Phe
       210                 215                 220
   Arg Leu Ile Ser Val Ser Ser Phe Cys Val Cys Asp Leu Ala Asn Asp
   225                 230                 235                 240
   Asn Ser Ile Glu Asn Thr Ser Leu Ala Gly Ser Asn Phe Gly Ile Val
                   245                 250                 255
   Asp Ser Leu Gly Glu Leu Glu Ala Leu Met Glu Arg Met Lys Arg Leu
               260                 265                 270
   Gln Glu Asp Arg Glu Asp Asp Glu Thr Thr Arg Glu Glu Met Thr Thr
           275                 280                 285
   Arg Phe Glu Lys Glu Lys Lys Glu Ser Leu Phe Val Val Pro Gly Glu
       290                 295                 300
   Thr Leu His Ala Phe Pro His Ser Asp Phe Glu Asp Asp Val Ile Val
   305                 310                 315                 320
   Thr Ala Asp Val Ser Arg Tyr Ile Glu Asp Pro Ser Phe Gly Tyr Glu
                   325                 330                 335
   Asp Phe Ala Arg Arg Gly Glu Glu His Leu Pro Thr Phe Arg Ala Gln
               340                 345                 350
   Asp Tyr Thr Trp Glu Asn His Gly Phe Ser Leu Val Asn Arg Leu Tyr
           355                 360                 365
   Ser Asp Ile Gly His Leu Leu Asp Glu Lys Phe Arg Met Val Tyr Asn
       370                 375                 380
   Leu Thr Tyr Asn Thr Met Ala Thr His Glu Asp Val Asp Thr Thr Thr
   385 390 395 400
   Leu Arg Arg Ala Leu Phe Asn Tyr Val His Cys Met Phe Gly Ile Arg
   405 410 415
   Tyr Asp Asp Tyr Asp Tyr Gly Glu Val Asn Gln Leu Leu Glu Arg Ser
   420 425 430
   Leu Lys Val Tyr Ile Lys Thr Val Thr Cys Tyr Pro Glu Arg Thr Thr
   435 440 445
   Lys Arg Met Tyr Asp Ser Tyr Trp Arg Gln Phe Thr His Ser Glu Lys
   450 455 460
   Val His Val Asn Leu Leu Leu Met Glu Ala Arg Met Gln Ala Glu Leu
   465 470 475 480
   Leu Tyr Ala Leu Arg Ala Ile Thr Arg His Leu Thr
   485 490
<210> 3
   <211> 480
   <212> PRT
   <213> Mus musculus
<400> 3
   Met Ile Val Ala Asp Ser Glu Cys His Ser Glu Ile Lys Gly Tyr Leu
   1               5                   10                  15
   Pro Phe Thr Arg Gly Gly Val Ala Gly Pro Glu Thr Arg Glu Glu His
               20                  25                  30
   Arg Glu Gly Gln Ala Arg Arg Gly Ser Arg Gly Pro Ser Ala Phe Ile
           35                  40                  45
   Pro Val Glu Glu Ile Leu Arg Glu Gly Ala Glu Ser Leu Glu Gln His
       50                  55                  60
   Leu Gly Leu Glu Ala Leu Met Ser Ser Gly Arg Val Asp Asn Leu Ala
   65                  70                  75                  80
   Val Val Met Gly Leu His Pro Asp Tyr Leu Ser Ser Phe Trp Arg Leu
                   85                  90                  95
   His Tyr Leu Leu Leu His Thr Asp Gly Pro Leu Ala Ser Ser Trp Arg
               100                 105                 110
   His Tyr Ile Ala Ile Met Ala Ala Ala Arg His Gln Cys Ser Tyr Leu
           115                 120                 125
   Val Gly Ser His Met Thr Glu Phe Leu Gln Thr Gly Gly Asp Pro Glu
       130                 135                 140
   Trp Leu Leu Gly Leu His Arg Ala Pro Glu Lys Leu Arg Lys Leu Ser
   145                 150                 155                 160
   Glu Val Asn Lys Leu Leu Ala His Arg Pro Trp Leu Ile Thr Lys Glu
                   165                 170                 175
   His Ile Gln Ala Leu Leu Lys Thr Gly Glu His Ser Trp Ser Leu Ala
               180                 185                 190
   Glu Leu Ile Gln Ala Leu Val Leu Leu Thr His Cys His Ser Leu Ala
           195                 200                 205
   Ser Phe Val Phe Gly Cys Gly Ile Leu Pro Glu Gly Asp Ala Glu Gly
       210                 215                 220
   Ser Pro Ala Ser Gln Ala Pro Ser Pro Pro Ser Glu Gln Gly Thr Pro
   225                 230                 235                 240
   Pro Ser Gly Asp Pro Leu Asn Asn Ser Gly Gly Phe Glu Ala Ala Arg
                   245                 250                 255
   Asp Val Glu Ala Leu Met Glu Arg Met Arg Gln Leu Gln Glu Ser Leu
               260                 265                 270
   Leu Arg Asp Glu Gly Ala Ser Gln Glu Glu Met Glu Asn Arg Phe Glu
           275                 280                 285
   Leu Glu Lys Ser Glu Ser Leu Leu Val Thr Pro Ser Ala Asp Ile Leu
       290                 295                 300
   Glu Pro Ser Pro His Pro Asp Ile Leu Cys Phe Val Glu Asp Pro Ala
   305                 310                 315                 320
   Phe Gly Tyr Glu Asp Phe Thr Arg Arg Gly Thr Gln Ala Pro Pro Thr
                   325                 330                 335
   Phe Arg Ala Gln Asp Tyr Thr Trp Glu Asp His Gly Tyr Ser Leu Ile
               340                 345                 350
   Gln Arg Leu Tyr Pro Glu Gly Gly Gln Leu Leu Asp Glu Lys Phe Gln
           355                 360                 365
   Val Ala Cys Ser Leu Thr Tyr Asn Thr Ile Ala Met His Ser Gly Val
       370                 375                 380
   Asp Thr Ser Met Leu Arg Arg Ala Ile Trp Asn Tyr Ile His Cys Val
   385                 390                 395                 400
   Phe Gly Ile Arg Tyr Asp Asp Tyr Asp Tyr Gly Glu Val Asn Gln Leu
                   405                 410                 415
   Leu Glu Arg Asn Leu Lys Ile Tyr Ile Lys Thr Val Ala Cys Tyr Pro
               420                 425                 430
   Glu Lys Thr Thr Arg Arg Met Tyr Asn Leu Phe Trp Arg His Phe Arg
           435                 440                 445
   His Ser Glu Lys Val His Val Asn Leu Leu Leu Leu Glu Ala Arg Met
       450                 455                 460
   Gln Ala Ala Leu Leu Tyr Ala Leu Arg Ala Ile Thr Arg Tyr Met Thr
   465                 470                 475                 480
<210> 4
   <211> 48
   <212> DNA
   <213> Artificial
<220>
   <223> primer
<220>
   <221> misc feature
   <222> (36)..(47)
   <223> n= inosine
<400> 4
   ctactactac taggccacgc gtcgactagt acgggnnggg nngggnng 48
<210> 5
   <211> 22
   <212> DNA
   <213> Artificial
<220>
   <223> primer
<400> 5
   gccagggttt tcccagtcac ga 22
<210> 6
   <211> 32
   <212> DNA
   <213> Artificial
<220>
   <223> primer
<400> 6
   ctactactac taggccacgc gtcgactagt ac 32
<210> 7
   <211> 38
   <212> DNA
   <213> Artificial
<220>
<223> primer
<400> 7
   tgtaaaacga cggccagtgt gaaggctgtg cgaggccg 38
<210> 8
   <211> 21
   <212> DNA
   <213> Artificial
<220>
   <223> primer
<400> 8
   cctggaacgg aacctcaaaa t 21
<210> 9
   <211> 20
   <212> DNA
   <213> Artificial
<220>
   <223> primer
<400> 9
   gggcttcaag gagcagcaag 20
<210> 10
   <211> 8950
   <212> DNA
   <213> Artificial
<220>
   <223> gene trap vector pTbetageo
<400> 10
   tgtgagcaaa aggccagcaa aaggccagga accgtaaaaa ggccgcgttg ctggcgtttt 60
   tccataggct ccgcccccct gacgagcatc acaaaaatcg acgctcaagt cagaggtggc 120
   gaaacccgac aggactataa agataccagg cgtttccccc tggaagctcc ctcgtgcgct 180
   ctcctgttcc gaccctgccg cttaccggat acctgtccgc ctttctccct tcgggaagcg 240
   tggcgctttc tcatagctca cgctgtaggt atctcagttc ggtgtaggtc gttcgctcca 300
   agctgggctg tgtgcacgaa ccccccgttc agcccgaccg ctgcgcctta tccggtaact 360
   atcgtcttga gtccaacccg gtaagacacg acttatcgcc actggcagca gccactggta 420
   acaggattag cagagcgagg tatgtaggcg gtgctacaga gttcttgaag tggtggccta 480
   actacggcta cactagaaga acagtatttg gtatctgcgc tctgctgaag ccagttacct 540
   tcggaaaaag agttggtagc tcttgatccg gcaaacaaac caccgctggt agcggtggtt 600
   tttttgtttg caagcagcag attacgcgca gaaaaaaagg atctcaagaa gatcctttga 660
   tcttttctac ggggtctgac gctcagtgga acgaaaactc acgttaaggg attttggtca 720
   tgagattatc aaaaaggatc ttcacctaga tccttttaaa ttaaaaatga agttttaaat 780
   caatctaaag tatatatgag taaacttggt ctgacagtta ccaatgctta atcagtgagg 840
   cacctatctc agcgatctgt ctatttcgtt catccatagt tgcctgactc cccgtcgtgt 900
   agataactac gatacgggag ggcttaccat ctggccccag tgctgcaatg ataccgcgag 960
   acccacgctc accggctcca gatttatcag caataaacca gccagccgga agggccgagc 1020
   gcagaagtgg tcctgcaact ttatccgcct ccatccagtc tattaattgt tgccgggaag 1080
   ctagagtaag tagttcgcca gttaatagtt tgcgcaacgt tgttgccatt gctacaggca 1140
   tcgtggtgtc acgctcgtcg tttggtatgg cttcattcag ctccggttcc caacgatcaa 1200
   ggcgagttac atgatccccc atgttgtgca aaaaagcggt tagctccttc ggtcctccga 1260
   tcgttgtcag aagtaagttg gccgcagtgt tatcactcat ggttatggca gcactgcata 1320
   attctcttac tgtcatgcca tccgtaagat gcttttctgt gactggtgag tactcaacca 1380
   agtcattctg agaatagtgt atgcggcgac cgagttgctc ttgcccggcg tcaatacggg 1440
   ataataccgc gccacatagc agaactttaa aagtgctcat cattggaaaa cgttcttcgg 1500
   ggcgaaaact ctcaaggatc ttaccgctgt tgagatccag ttcgatgtaa cccactcgtg 1560
   cacccaactg atcttcagca tcttttactt tcaccagcgt ttctgggtga gcaaaaacag 1620
   gaaggcaaaa tgccgcaaaa aagggaataa gggcgacacg gaaatgttga atactcatac 1680
   tcttcctttt tcaatattat tgaagcattt atcagggtta ttgtctcatg agcggataca 1740
   tatttgaatg tatttagaaa aataaacaaa taggggttcc gcgcacattt ccccgaaaag 1800
   tgccacctga cgtctaagaa accattatta tcatgacatt aacctataaa aataggcgta 1860
   tcacgaggcc ctttcgtctc gcgcgtttcg gtgatgacgg tgaaaacctc tgacacatgc 1920
   agctcccgga gacggtcaca gcttgtctgt aagcggatgc cgggagcaga caagcccgtc 1980
   agggcgcgtc agcgggtgtt ggcgggtgtc ggggctggct taactatgcg gcatcagagc 2040
   agattgtact gagagtgcac catatgcggt gtgaaatacc gcacagatgc gtaaggagaa 2100
   aataccgcat caggcgccat tcgccattca ggctgcgcaa ctgttgggaa gggcgatcgg 2160
   tgcgggcctc ttcgctatta cgccagctgg cgaaaggggg atgtgctgca aggcgattaa 2220
   gttgggtaac gccagggttt tcccagtcac gacgttgtaa aacgacggcc agtgccaagc 2280
   ttggaattca tgggaagagg aaccgaaagt atgtttttca gatgttcttt ctcagaaata 2340
   ggagtttgcg gaggttggag tgtgtgttgt aggacacgaa ccccagggtg gaggagactg 2400
   gaggacagag ccctctttcc cagggaggga aggaggagag tttgagatcc gctccggaag 2460
   tcggggttca ggtttgagca ggccaggcct ctcccgtggt ctcgccctct tgtcctagaa 2520
   gcctcactgg ccaggtgtaa gccaggtcgt gggtgccgag ccctgctccc tcatcctcag 2580
   catggatgtg aagaggactg tatggcgtgc gggtgtgtgt gaccgtgggt acacttaaaa 2640
   caccgggttt tggatctgca ctgtcccgga tgtcctctgg tgctcaaaga cccttttggg 2700
   tttgcccttt ggtaagagcg ccgggatcta cttgtctgga ggccagggag tcctcagccg 2760
   aggcttgccg cccctgactg cactgcactg agtagtggat gggagagtct ggtaccgcac 2820
   tgccggtttc ctccaccatc cccgcagcgc agggcagtgc attccgtcct ggctgcgaag 2880
   ggggatggtc gggccttctc cagcctcttc cgcttctagc ggaaggggcc ttgatggaag 2940
   ggcccgcatg tctccaaagt tgattcatgc ttcttgcaca gagaaagacc agaaagaagg 3000
   tctcaagttt tagccggtag cccggatggc ctttcctgca cggcaccata tgaaccttgt 3060
   gaccctgaca aagagacccc tctaacccaa gacccctacc acttaccctt tccctttgaa 3120
   ggctttccca caccaccctc cacacttgcc ccaaacactg ccaactatgt aggaggaagg 3180
   ggttgggact aacagaagaa cccgttgtgg ggaagctgtt gggagggtca ctttatgttc 3240
   ttgcccaagg tcagttgggt ggcctgcttc tgatgaggtg gtcccaaggt ctggggtaga 3300
   aggtgagagg gacaggccac caaggtcagc cccccccccc tatcccatag gagccaggtc 3360
   cctctcctgg acaggaagac tgaaggggag atgccagaga ctcagtgaag cctggggtac 3420
   cctattggag tccttcaagg aaacaaactt ggcctcacca ggcctcagcc ttggctcctc 3480
   ctgggaactc tactgccctt gggatcccct tgtagttgtg ggttacatag gaagggggac 3540
   gggattcccc ttgactggct agcctactct tttcttcagt cttctccatc tcctctcacc 3600
   tgtctctcga ccctttccct aggatagact tggaaaaaga taaggggaga aaacaaatgc 3660
   aaacgaggcc agaaagattt tggctgggca ttccttccgc tagcttttat tgggatcccc 3720
   tagtttgtga taggcctttt agctacatct gccaatccat ctcattttca cacacacaca 3780
   caccactttc cttctggtca gtgggcacat gtccagcctc aagtttatat caccaccccc 3840
   aatgcccaac acttgtatgg ccttgggcgg gtcatccccc cccccacccc cagtatctgc 3900
   aacctcaagc tagcttgggt gcgttggttg tggataagta gctagactcc agcaaccagt 3960
   aacctctgcc ctttctcctc catgacaacc aggtcccagg tcccgaaaac caaagaagaa 4020
   gaaccctaac aaagaggaca agcggcctcg cacagccttc actgctgagc agctccagag 4080
   gctcaaggct gagtttcaga ccaacaggta cctgacagag cagcggcgcc agagtctggc 4140
   acaggagctc ggtacccgga agatctggac tctagaggat cccgtcgttt tacaacgtcg 4200
   tgactgggaa aaccctggcg ttacccaact taatcgcctt gcagcacatc ccccttccgc 4260
   cagctggcgt aatagcgaag aggcccgcac cgatcgccct tcccaacagt tgcgcagcct 4320
   gaatggcgaa tggcgctttg cctggtttcc ggcaccagaa gcggtgccgg aaagctggct 4380
   ggagtgcgat cttcctgagg ccgatactgt cgtcgtcccc tcaaactggc agatgcacgg 4440
   ttacgatgcg cccatctaca ccaacgtgac ctatcccatt acggtcaatc cgccgtttgt 4500
   tcccacggag aatccgacgg gttgttactc gctcacattt aatgttgatg aaagctggct 4560
   acaggaaggc cagacgcgaa ttatttttga tggcgttaac tcggcgtttc atctgtggtg 4620
   caacgggcgc tgggtcggtt acggccagga cagtcgtttg ccgtctgaat ttgacctgag 4680
   cgcattttta cgcgccggag aaaaccgcct cgcggtgatg gtgctgcgct ggagtgacgg 4740
   cagttatctg gaagatcagg atatgtggcg gatgagcggc attttccgtg acgtctcgtt 4800
   gctgcataaa ccgactacac aaatcagcga tttccatgtt gccactcgct ttaatgatga 4860
   tttcagccgc gctgtactgg aggctgaagt tcagatgtgc ggcgagttgc gtgactacct 4920
   acgggtaaca gtttctttat ggcagggtga aacgcaggtc gccagcggca ccgcgccttt 4980
   cggcggtgaa attatcgatg agcgtggtgg ttatgccgat cgcgtcacac tacgtctgaa 5040
   cgtcgaaaac ccgaaactgt ggagcgccga aatcccgaat ctctatcgtg cggtggttga 5100
   actgcacacc gccgacggca cgctgattga agcagaagcc tgcgatgtcg gtttccgcga 5160
   ggtgcggatt gaaaatggtc tgctgctgct gaacggcaag ccgttgctga ttcgaggcgt 5220
   taaccgtcac gagcatcatc ctctgcatgg tcaggtcatg gatgagcaga cgatggtgca 5280
   ggatatcctg ctgatgaagc agaacaactt taacgccgtg cgctgttcgc attatccgaa 5340
   ccatccgctg tggtacacgc tgtgcgaccg ctacggcctg tatgtggtgg atgaagccaa 5400
   tattgaaacc cacggcatgg tgccaatgaa tcgtctgacc gatgatccgc gctggctacc 5460
   ggcgatgagc gaacgcgtaa cgcgaatggt gcagcgcgat cgtaatcacc cgagtgtgat 5520
   catctggtcg ctggggaatg aatcaggcca cggcgctaat cacgacgcgc tgtatcgctg 5580
   gatcaaatct gtcgatcctt cccgcccggt gcagtatgaa ggcggcggag ccgacaccac 5640
   ggccaccgat attatttgcc cgatgtacgc gcgcgtggat gaagaccagc ccttcccggc 5700
   tgtgccgaaa tggtccatca aaaaatggct ttcgctacct ggagagacgc gcccgctgat 5760
   cctttgcgaa tacgcccacg cgatgggtaa cagtcttggc ggtttcgcta aatactggca 5820
   ggcgtttcgt cagtatcccc gtttacaggg cggcttcgtc tgggactggg tggatcagtc 5880
   gctgattaaa tatgatgaaa acggcaaccc gtggtcggct tacggcggtg attttggcga 5940
   tacgccgaac gatcgccagt tctgtatgaa cggtctggtc tttgccgacc gcacgccgca 6000
   tccagcgctg acggaagcaa aacaccagca gcagtttttc cagttccgtt tatccgggca   6060
   aaccatcgaa gtgaccagcg aatacctgtt ccgtcatagc gataacgagc tcctgcactg   6120
   gatggtggcg ctggatggta agccgctggc aagcggtgaa gtgcctctgg atgtcgctcc   6180
   acaaggtaaa cagttgattg aactgcctga actaccgcag ccggagagcg ccgggcaact   6240
   ctggctcaca gtacgcgtag tgcaaccgaa cgcgaccgca tggtcagaag ccgggcacat   6300
   cagcgcctgg cagcagtggc gtctggcgga aaacctcagt gtgacgctcc ccgccgcgtc   6360
   ccacgccatc ccgcatctga ccaccagcga aatggatttt tgcatcgagc tgggtaataa   6420
   gcgttggcaa tttaaccgcc agtcaggctt tctttcacag atgtggattg gcgataaaaa   6480
   acaactgctg acgccgctgc gcgatcagtt cacccgtgca ccgctggata acgacattgg   6540
   cgtaagtgaa gcgacccgca ttgaccctaa cgcctgggtc gaacgctgga aggcggcggg   6600
   ccattaccag gccgaagcag cgttgttgca gtgcacggca gatacacttg ctgatgcggt   6660
   gctgattacg accgctcacg cgtggcagca tcaggggaaa accttattta tcagccggaa   6720
   aacctaccgg attgatggta gtggtcaaat ggcgattacc gttgatgttg aagtggcgag   6780
   cgatacaccg catccggcgc ggattggcct gaactgccag ctggcgcagg tagcagagcg   6840
   ggtaaactgg ctcggattag ggccgcaaga aaactatccc gaccgcctta ctgccgcctg   6900
   ttttgaccgc tgggatctgc cattgtcaga catgtatacc ccgtacgtct tcccgagcga   6960
   aaacggtctg cgctgcggga cgcgcgaatt gaattatggc ccacaccagt ggcgcggcga   7020
   cttccagttc aacatcagcc gctacagtca acagcaactg atggaaacca gccatcgcca   7080
   tctgctgcac gcggaagaag gcacatggct gaatatcgac ggtttccata tggggattgg   7140
   tggcgacgac tcctggagcc cgtcagtatc ggcggaattc cagctgagcg ccggtcgcta   7200
   ccattaccag ttggtctggt gtcaggggat cccccgggct gcagccaata tgggatcggc   7260
   cattgaacaa gatggattgc acgcaggttc tccggccgct tgggtggaga ggctattcgg   7320
   ctatgactgg gcacaacaga caatcggctg ctctgatgcc gccgtgttcc ggctgtcagc   7380
   gcaggggcgc ccggttcttt ttgtcaagac cgacctgtcc ggtgccctga atgaactgca   7440
   ggacgaggca gcgcggctat cgtggctggc cacgacgggc gttccttgcg cagctgtgct   7500
   cgacgttgtc actgaagcgg gaagggactg gctgctattg ggcgaagtgc cggggcagga   7560
   tctcctgtca tctcaccttg ctcctgccga gaaagtatcc atcatggctg atgcaatgcg   7620
   gcggctgcat acgcttgatc cggctacctg cccattcgac caccaagcga aacatcgcat   7680
   cgagcgagca cgtactcgga tggaagccgg tcttgtcgat caggatgatc tggacgaaga   7740
   gcatcagggg ctcgcgccag ccgaactgtt cgccaggctc aaggcgcgca tgcccgacgg   7800
   cgaggatctc gtcgtgaccc atggcgatgc ctgcttgccg aatatcatgg tggaaaatgg   7860
   ccgcttttct ggattcatcg actgtggccg gctgggtgtg gcggaccgct atcaggacat 7920
   agcgttggct acccgtgata ttgctgaaga gcttggcggc gaatgggctg accgcttcct 7980
   cgtgctttac ggtatcgccg ctcccgattc gcagcgcatc gccttctatc gccttcttga 8040
   cgagttcttc tgagcgggac tctggggttc gaaatgaccg accaagcgac gcccaacctg 8100
   ccatcacgag atttcgattc caccgccgcc ttctatgaaa ggttgggctt cggaatcgtt 8160
   ttccgggacg ccggctggat gatcctccag cgcggggatc tcatgctgga gttcttcgcc 8220
   caccccccgg atctaagctc tagataagta atgatcataa tcagccatat cacatctgta 8280
   gaggttttac ttgctttaaa aaacctccca cacctccccc tgaacctgaa acataaaatg 8340
   aatgcaattg ttgttgttaa cttgtttatt gcagcttata atggttacaa ataaagcaat 8400
   agcatcacaa atttcacaaa taaagcattt ttttcactgc attctagttg tggtttgtcc 8460
   aaactcatca atgtatctta tcatgtctgg atccgggggt accgagctcg aattcgtaat 8520
   catgtcatag ctgtttcctg tgtgaaattg ttatccgctc acaattccac acaacatacg 8580
   agccggaagc ataaagtgta aagcctgggg tgcctaatga gtgagctaac tcacattaat 8640
   tgcgttgcgc tcactgcccg ctttccagtc gggaaacctg tcgtgccagc tgcattaatg 8700
   aatcggccaa cgcgcgggga gaggcggttt gcgtattggg cgctcttccg cttcctcgct 8760
   cactgactcg ctgcgctcgg tcgttcggct gcggcgagcg gtatcagctc actcaaaggc 8820
   ggtaatacgg ttatccacag aatcagggga taacgcagga aagaacatgt gagcaaaagg 8880
   ccagcaaaag gccaggaacc gtaaaaaggc cgcgttgctg gcgtttttcc ataggctccg 8940
   cccccctgac 8950
<210> 11
   <211> 20
   <212> DNA
   <213> Artificial
<220>
   <223> primer
<400> 11
   cagccttgag cctctggagc 20
<210> 12
   <211> 21
   <212> DNA
   <213> Artificial
<220>
   <223> primer
<400> 12
   ctaccctgag aagacgaccc g 21
<210> 13
   <211> 21
   <212> DNA
   <213> Artificial
<220>
   <223> primer
<400> 13
   ctaccctgag aagacgaccc g 21
<210> 14
   <211> 30
   <212> DNA
   <213> Artificial
<220>
   <223> primer
<400> 14
   ggacaaatca aggttacaca gaaaaaagtc 30
<210> 15
   <211> 20
   <212> DNA
   <213> Artificial
<220>
   <223> primer
<400> 15
   cagccttgag cctctggagc 20
<210> 16
   <211> 4520
   <212> DNA
   <213> Artificial
<220>
   <223> gene trap vector U3Cre
<400> 16
   aatgaaagac cccacctgta ggtttggcaa gctagcatgc ccaagaagaa gaggaaggtg     60
   tccaatttac tgaccgtaca ccaaaatttg cctgcattac cggtcgatgc aacgagtgat    120
   gaggttcgca agaacctgat ggacatgttc agggatcgcc aggcgttttc tgagcatacc    180
   tggaaaatgc ttctgtccgt ttgccggtcg tgggcggcat ggtgcaagtt gaataaccgg    240
   aaatggtttc ccgcagaacc tgaagatgtt cgcgattatc ttctatatct tcaggcgcgc    300
   ggtctggcag taaaaactat ccagcaacat ttgggccagc taaacatgct tcatcgtcgg    360
   tccgggctgc cacgaccaag tgacagcaat gctgtttcac tggttatgcg gcggatccga    420
   aaagaaaacg ttgatgccgg tgaacgtgca aaacaggctc tagcgttcga acgcactgat    480
   ttcgaccagg ttcgttcact catggaaaat agcgatcgct gccaggatat acgtaatctg    540
   gcatttctgg ggattgctta taacaccctg ttacgtatag ccgaaattgc caggatcagg 600
   gttaaagata tctcacgtac tgacggtggg agaatgttaa tccatattgg cagaacgaaa 660
   acgctggtta gcaccgcagg tgtagagaag gcacttagcc tgggggtaac taaactggtc 720
   gagcgatgga tttccgtctc tggtgtagct gatgatccga ataactacct gttttgccgg 780
   gtcagaaaaa atggtgttgc cgcgccatct gccaccagcc agctatcaac tcgcgccctg 840
   gaagggattt ttgaagcaac tcatcgattg atttacggcg ctaaggatga ctctggtcag 900
   agatacctgg cctggtctgg acacagtgcc cgtgtcggag ccgcgcgaga tatggcccgc 960
   gctggagttt caataccgga gatcatgcaa gctggtggct ggaccaatgt aaatattgtc 1020
   atgaactata tccgtaacct ggatagtgaa acaggggcaa tggtgcgcct gctggaagat 1080
   ggcgattagg ctagcttaag taacgccatt ttgcaaggca tggaaaaata cataactgag 1140
   aatagagaag ttcagatcaa ggtcaggaac agatggaaca gctagagaac catcagatgt 1200
   ttccagggtg ccccaaggac ctgaaatgac cctgtgcctt atttgaacta accaatcagt 1260
   tcgcttctcg cttctgttcg cgcgcttctg ctccccgagc tcaataaaag agcccacaac 1320
   ccctcactcg gggcgccagt cctccgattg actgagtcgc ccgggtaccc gtgtatccaa 1380
   taaaccctct tgcagttgca tccgacttgt ggtctcgctg ttccttggga gggtctcctc 1440
   tgagtgattg actacccgtc agcgggggtc tttcatttgg gggctcgtcc gggatcggga 1500
   gacccctgcc cagggaccac cgacccacca ccgggaggta agctggccag caacttatct 1560
   gtgtctgtcc gattgtctag tgtctatgac tgattttatg cgcctgcgtc ggtactagtt 1620
   agctaactag ctctgtatct ggcggacccg tggtggaact gacgagttcg gaacacccgg 1680
   ccgcaaccct gggagacgtc ccagggactt cgggggccgt ttttgtggcc cgacctgagt 1740
   ccaaaaatcc cgatcgtttt ggactctttg gtgcaccccc cttagaggag ggatatgtgg 1800
   ttctggtagg agacgagaac ctaaaacagt tcccgcctcc gtctgaattt ttgctttcgg 1860
   tttgggaccg aagccgcgcc gcgcgtcttg tctgctgcag catcgttctg tgttgtctct 1920
   gtctgactgt gtttctgtat ttgtctgaga atatgggcca gactgttacc actcccttaa 1980
   gtttgacctt aggtcactgg aaagatgtcg agcggatcgc tcacaaccag tcggtagatg 2040
   tcaagaagag acgttgggtt accttctgct ctgcagaatg gccaaccttt aacgtcggat 2100
   ggccgcgaga cggcaccttt aaccgagacc tcatcaccca ggttaagatc aaggtctttt 2160
   cacctggccc gcatggacac ccagaccagg tcccctacat cgtgacctgg gaagccttgg 2220
   cttttgaccc ccctccctgg gtcaagccct ttgtacaccc taagcctccg cctcctcttc 2280
   ctccatccgc cccgtctctc ccccttgaac ctcctcgttc gaccccgcct cgatcctccc 2340
   tttatccagc cctcactcct tctctaggcg ccaaacctaa acctcaagtt ctttctgaca 2400
   gtgggggccc gctcatcgac ctacttacag aagacccccc gccttatagg gacccaagac 2460
   cacccccttc cgacagggac ggaaatggtg gagaagcgac ccctgcggga gaggcaccgg 2520
   acccctcccc aattgcatct cgcctacgtg ggagacggga gccccctgtg gccgactcca 2580
   ctacctcgca ggcattcccc ctccgcgcag gaggaaacgg acagcttcaa tactggccgt 2640
   tctcctcttc tgacctttac aactggaaaa ataataaccc ttctttttct gaagatccag 2700
   gtaaactgac agctctgatc gagtctgttc tcatcaccca tcagcccacc tgggacgact 2760
   gtcagcagct gttggggact ctgctgaccg gagaagaaaa acaacgggtg ctcttagagg 2820
   ctagaaaggc ggtgcggggc gatgatgggc gccccactca actgcccaat gaagtcgatg 2880
   ccgcttttcc cctcgaggga tccatcgtag tccaatttgt taaagacagg atatcagtgg 2940
   tccaggctct agttttgact caacaatatc accagctgaa gcctatagag tacgagccat 3000
   agataaaata aaagatttta tttagtctcc agaaaaaggg gggaatgaaa gaccccacct 3060
   gtaggtttgg caagctagca tgcccaagaa gaagaggaag gtgtccaatt tactgaccgt 3120
   acaccaaaat ttgcctgcat taccggtcga tgcaacgagt gatgaggttc gcaagaacct 3180
   gatggacatg ttcagggatc gccaggcgtt ttctgagcat acctggaaaa tgcttctgtc 3240
   cgtttgccgg tcgtgggcgg catggtgcaa gttgaataac cggaaatggt ttcccgcaga 3300
   acctgaagat gttcgcgatt atcttctata tcttcaggcg cgcggtctgg cagtaaaaac 3360
   tatccagcaa catttgggcc agctaaacat gcttcatcgt cggtccgggc tgccacgacc 3420
   aagtgacagc aatgctgttt cactggttat gcggcggatc cgaaaagaaa acgttgatgc 3480
   cggtgaacgt gcaaaacagg ctctagcgtt cgaacgcact gatttcgacc aggttcgttc 3540
   actcatggaa aatagcgatc gctgccagga tatacgtaat ctggcatttc tggggattgc 3600
   ttataacacc ctgttacgta tagccgaaat tgccaggatc agggttaaag atatctcacg 3660
   tactgacggt gggagaatgt taatccatat tggcagaacg aaaacgctgg ttagcaccgc 3720
   aggtgtagag aaggcactta gcctgggggt aactaaactg gtcgagcgat ggatttccgt 3780
   ctctggtgta gctgatgatc cgaataacta cctgttttgc cgggtcagaa aaaatggtgt 3840
   tgccgcgcca tctgccacca gccagctatc aactcgcgcc ctggaaggga tttttgaagc 3900
   aactcatcga ttgatttacg gcgctaagga tgactctggt cagagatacc tggcctggtc 3960
   tggacacagt gcccgtgtcg gagccgcgcg agatatggcc cgcgctggag tttcaatacc 4020
   ggagatcatg caagctggtg gctggaccaa tgtaaatatt gtcatgaact atatccgtaa 4080
   cctggatagt gaaacagggg caatggtgcg cctgctggaa gatggcgatt aggctagctt 4140
   aagtaacgcc attttgcaag gcatggaaaa atacataact gagaatagag aagttcagat 4200
   caaggtcagg aacagatgga acagctagag aaccatcaga tgtttccagg gtgccccaag 4260
   gacctgaaat gaccctgtgc cttatttgaa ctaaccaatc agttcgcttc tcgcttctgt 4320
   tcgcgcgctt ctgctccccg agctcaataa aagagcccac aacccctcac tcggggcgcc 4380
   agtcctccga ttgactgagt cgcccgggta cccgtgtatc caataaaccc tcttgcagtt 4440
   gcatccgact tgtggtctcg ctgttccttg ggagggtctc ctctgagtga ttgactaccc 4500
   gtcagcgggg gtctttcatt 4520
<210> 17
<211> 23
   <212> DNA
   <213> Artificial
<220>
   <223> primer
<400> 17
   ccaatcttgc ttctttgctg agc 23
<210> 18
   <211> 22
   <212> DNA
   <213> Artificial
<220>
   <223> primer
<400> 18
   ggctcatgct tgaatgtttc ag 22
<210> 19
   <211> 23
   <212> DNA
   <213> Artificial
<220>
   <223> primer
<400> 19
   ccaatcttgc ttctttgctg agc 23
<210> 20
   <211> 20
   <212> DNA
   <213> Artificial
<220>
   <223> primer
<400> 20
   atcatgcaag ctggtggctg 20
<210> 21
   <211> 21
   <212> DNA
   <213> Artificial
<220>
   <223> primer
<400> 21
   ctaccctgag aagacgaccc g 21
<210> 22
   <211> 30
   <212> DNA
   <213> Artificial
<220>
   <223> primer
<400> 22
   ggacaaatca aggttacaca gaaaaaagtc 30
<210> 23
   <211> 21
   <212> DNA
   <213> Artificial
<220>
   <223> primer
<400> 23
   ctaccctgag aagacgaccc g 21
<210> 24
   <211> 20
   <212> DNA
   <213> Artificial
<220>
   <223> primer
<400> 24
   cagccttgag cctctggagc 20